# EUROPEAN PATENT SPECIFICATION

(11) **EP 4 438 590 B1**
(45) Date of publication and mention of the grant of the patent: **21.10.2026**
(21) Application number: 22898626.1
(22) Date of filing: 24.11.2022
(51) Int. Cl.: H10K 50/18, C07F 7/08, C09K 11/06, C07C 211/54, C07D 209/86, C07D 307/91, C07D 333/76, C07D 405/12, H10K 50/15, H10K 85/60

(54) **ARYLAMINE COMPOUND, ORGANIC ELECTROLUMINESCENT ELEMENT, AND ELECTRONIC DEVICE**
ARYLAMINVERBINDUNG, ORGANISCHES ELEKTROLUMINESZENZELEMENT UND ELEKTRONISCHE VORRICHTUNG
COMPOSÉ ARYLAMINE, ÉLÉMENT ÉLECTROLUMINESCENT ORGANIQUE ET DISPOSITIF ÉLECTRONIQUE

(30) Priority: 25.11.2021 JP 2021190664
(43) Date of publication of application: 02.10.2024
(73) Proprietor: Hodogaya Chemical Co., Ltd., Tokyo, 105-0021 (JP)
(72) Inventor: KASE, Kouki, Tokyo 105-0021 (JP); LIM, Jae-Geon, Tokyo 105-0021 (JP); SHIN, Heung-Seob, Tokyo 105-0021 (JP); CHA, Hyun-Wook, Tokyo 105-0021 (JP); IZUMIDA, Junichi, Tokyo 105-0021 (JP); HAYASHI, Shuichi, Tokyo 105-0021 (JP)
(74) Representative: Müller-Boré & Partner Patentanwälte PartG mbB
(86) International application number: PCT/JP2022/043412
(87) International publication number: WO 2023/095844

(56) References cited:
- WO-A1-2021/054714
- WO-A1-2021/054714
- WO-A1-2021/132995
- WO-A1-2021/132995
- WO-A1-2022/134602
- WO-A1-2022/154283
- CN-A- 112 830 889
- CN-A- 112 830 889

## Description

### Technical Field

The present invention relates to compounds and devices suitable for an organic electroluminescent device (hereinafter referred to as organic EL device), which is a preferred self-luminous device for various display devices, and more specifically relates to an arylamine compound and an organic EL device using the compound.

### Background Art

The organic EL device is a self-luminous device and has been actively studied for their brighter, superior visibility and the ability to display clearer images in comparison with liquid crystal devices.

In 1987, C. W. Tang and colleagues at Eastman Kodak developed a laminated structure device using materials assigned with different roles, realizing practical applications of an organic EL device with organic materials. These researchers laminated an electron-transporting phosphor and a hole-transporting organic substance, and injected both charges into a phosphor layer to cause emission in order to obtain a high luminance of 1,000 cd/m² or more at a voltage of 10 V or less (refer to Patent Documents 1 and 2, for example).

To date, various improvements have been made for practical applications of the organic EL device. Various roles of the laminated structure are further subdivided to provide an EL device that includes an anode, a hole injection layer, a hole transport layer, a light emitting layer, an electron transport layer, an electron injection layer, and a cathode successively formed on a substrate, and high efficiency and durability have been achieved by the EL device (refer to Non-Patent Document 1, for example).

Further, there have been attempts to use triplet excitons for further improvements of luminous efficiency, and the use of a phosphorescence-emitting compound has been examined (refer to Non-Patent Document 2, for example).

Devices that use light emission caused by thermally activated delayed fluorescence (TADF) have also been developed. In 2011, Adachi et al. at Kyushu University, National University Corporation realized 5.3% external quantum efficiency with a device using a thermally activated delayed fluorescent material (refer to Non-Patent Document 3, for example).

The light emitting layer can be also fabricated by doping a charge-transporting compound generally called a host material, with a fluorescent compound, a phosphorescence-emitting compound, or a delayed fluorescent-emitting material. As described in the Non-Patent Document, the selection of organic materials in an organic EL device greatly influences various device characteristics such as efficiency and durability (refer to Non-Patent Documents 1 to 3, for example).

In an organic EL device, charges injected from both electrodes recombine in a light emitting layer to cause emission. What is important here is how efficiently the hole and electron charges are transferred to the light emitting layer in order to form a device having excellent carrier balance. Therefore, by using a material with characteristics that improving hole injectability, which provide the hole injected from an anode into the light emitting layer, and electron blocking performance, which block the electron injected from a cathode, the probability of hole-electron recombination in the light emitting layer can be improved. Furthermore, high luminous efficiency can be obtained by confining excitons generated in the light emitting layer. The role of a hole transport material is therefore important, and there is a need for a hole transport material that has high hole injectability, high hole mobility, high electron blocking performance, and high durability to the electron.

Heat resistance and amorphousness of the materials are also important with respect to the lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (NPD) and various aromatic amine derivatives are known as the hole transport materials used for the organic EL device (refer to Patent Documents 1 and **2,** for example). Although NPD has desirable hole transportability, its glass transition point (Tg), which is an index of heat resistance, is as low as 96°C, which causes the degradation of device characteristics by crystallization under a high-temperature condition (refer to Non-Patent Document 4, for example).

The aromatic amine derivatives described in the Patent Documents include a compound known to have an excellent hole mobility of 10⁻³ cm²/Vs or higher (refer to Patent Documents 1 and 2, for example). However, since the compound is insufficient in terms of electron blocking performance, some of the electrons pass through the light emitting layer, and improvements in luminous efficiency cannot be expected. For such a reason, a material with higher electron blocking performance, a more stable thin-film state, and higher heat resistance is needed for higher efficiency. Although an aromatic amine derivative having high durability is reported (refer to Patent Document 3, for example), the derivative is used as a charge transporting material used in an electrophotographic photoconductor, and there is no example of using the derivative in the organic EL device.

To solve this problem, a substituted carbazole structure and arylamine compounds are proposed as compounds improved in the characteristics such as heat resistance and hole injectability (refer to Patent Documents 4 and 5, for example). However, while the devices using these compounds for the hole injection layer or the hole transport layer have been improved in device lifetime, luminous efficiency, and the like, the improvements are still insufficient. Further lower driving voltage, higher luminous efficiency, and longer lifetime of devices are therefore needed.

Further arylamine compounds are known from Patent Documents 9 to 11, for instance.

### Citation List

### Patent Documents

Patent Document 1: US Patent No, 5,792,557
Patent Document 2: US Patent No, 5,639,914
Patent Document 3: US Patent No, 7,759,030
Patent Document 4: JP-A-2009-076817
Patent Document 5: Japanese Patent No. 6674892
Patent Document 6: EP Patent No. 2684932
Patent Document 7: WO2021/05471
Patent Document 8: WO2021/121230
Patent Document 9: WO 2021/054714 A1
Patent Document 10: CN 112 830 889 A
Patent Document 11: WO 2021/132995 A1

### Non-Patent Documents

Non-Patent Document 1: The Japan Society of Applied Physics, 9th Lecture Preprints, pp. 55 to 61 (2001)
Non-Patent Document 2: The Japan Society of Applied Physics, 9th Lecture Preprints, pp. 23 to 31 (2001)
Non-Patent Document 3: Appl. Phys. Let., 98, 083302 (2011)
Non-Patent Document 4: Organic EL Symposium, the 3rd Regular presentation Preprints, pp. 13 to 14 (2006)

### Summary of the Invention

### Technical Problem

An object of the present invention is to provide a material for an organic EL device having (1) excellent hole injection and transport abilities, (2) electron blocking ability, (3) high thin-film stability, and (4) excellent durability as a material for an organic EL device with high efficiency and high durability.

Further, an object of the present invention is to provide, by using the material of the present invention, an organic EL device having (1) high luminous efficiency and high-power efficiency, (2) low turn on voltage and low actual driving voltage, and (3) a long lifetime.

### Solution to Problem

For achieving the object, the present inventors have focused on the fact that an arylamine material is excellent in hole injection/transport ability, thin film stability, and durability, and by pursuing the optimization of a substitution position and a substituent of a naphthylene group, they were able to improve the characteristics of the materials drastically. In the organic EL device, the performance of luminous efficiency and power efficiency has been improved, turn on voltage and actual driving voltage can be suppressed, and a longer lifetime than the conventional lifetime can be achieved, therefore they have completed the invention.

Specifically, according to the present invention, the following arylamine compounds and organic EL devices and electronic apparatus using the compounds are provided.
1) An arylamine compound of the following general formula (1) or (2): 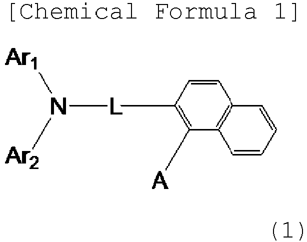 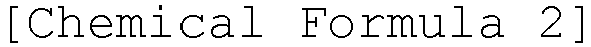 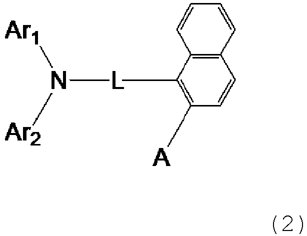 In the general formula (1) or (2), Ar₁ and Ar₂ may be the same or different, and represent a substituted or unsubstituted aromatic hydrocarbon group, a substituted or unsubstituted aromatic heterocyclic group, or a substituted or unsubstituted condensed polycyclic aromatic group. L represents a substituted or unsubstituted divalent group of an aromatic hydrocarbon, a substituted or unsubstituted divalent group of an aromatic heterocyclic ring, or a substituted or unsubstituted divalent group of condensed polycyclic aromatics. A represents a substituted or unsubstituted naphthyl group, a substituted or unsubstituted dibenzofuranyl group, a substituted or unsubstituted dibenzothienyl group, a substituted or unsubstituted carbazolyl group, a substituted or unsubstituted triphenylsilyl group, or a substituted or unsubstituted phenanthrenyl group.
2) The arylamine compound of 1), wherein, in the general formula (1) or (2), Ar₁ and/or Ar₂ is a substituted or unsubstituted phenyl group, a substituted or unsubstituted biphenyl group, a substituted or unsubstituted naphthyl group, a substituted or unsubstituted dibenzofuranyl group, a substituted or unsubstituted dibenzothienyl group, or a substituted or unsubstituted phenanthrenyl group.
3) The arylamine compound of 1) or 2), wherein, in the general formula (1) or (2), L is an unsubstituted phenylene group, an unsubstituted naphthylene group, or an unsubstituted biphenylene group.
4) The arylamine compound of any one of 1) to 3), wherein, in the general formula (1) or (2), A is an unsubstituted naphthyl group, an unsubstituted dibenzofuranyl group, an unsubstituted dibenzothienyl group, an unsubstituted carbazolyl group, an unsubstituted triphenylsilyl group, or an unsubstituted phenanthrenyl group.
5) The arylamine compound of any one of 1) to 4), wherein, in the general formula (1) or (2), A is an unsubstituted 1-naphthyl group, an unsubstituted 2-naphthyl group, an unsubstituted 1-dibenzofuranyl group, an unsubstituted 2-dibenzofuranyl group, an unsubstituted **3-**dibenzofuranyl group, an unsubstituted 4-dibenzofuranyl group, an unsubstituted 3-dibenzothienyl group, an unsubstituted 4-dibenzothienyl group, an unsubstituted 9-carbazolyl group, or an unsubstituted 9-phenanthrenyl group
6) An organic EL device including a pair of electrodes and at least one organic layer sandwiched therebetween, wherein the organic layer includes the arylamine compound of any one of the above 1) to 5).
7) The organic EL device of 6), wherein the organic layer is a hole transport layer.
8) The organic EL device of 6), wherein the organic layer is an electron blocking layer.
9) The organic EL device of 6), wherein the organic layer is a hole injection layer.
10) The organic EL device of 6), wherein the organic layer is a light emitting layer.
11) An electronic apparatus including a pair of electrodes and at least one organic layer sandwiched therebetween, wherein the organic layer includes the arylamine compound of any one of the above 1) to 5).

Specific examples of the "aromatic hydrocarbon group", the "aromatic heterocyclic ring group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ and Ar₂ in the general formulas (1) and (2) include a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, an azafluorenyl group, a diazafluorenyl group, an azaspirobifluorenyl group, a diazaspirobifluorenyl group, a quinoxalinyl group, a benzoimidazolyl group, a pyrazolyl group, a dibenzofuranyl group, a dibenzothienyl group, a naphthyridinyl group, a phenanthrolinyl group, an acridinyl group, and a carbolinyl group, and an aryl group of 6 to 30 carbon atoms, or a heteroaryl group of 2 to 20 carbon atoms, or the like may be selected.

Specific examples of the "substituent" in the "substituted aromatic hydrocarbon group", the "substituted aromatic heterocyclic ring group", or the "substituted condensed polycyclic aromatic group" represented by Ar₁ and Ar₂ in the general formulas (1) and (2) include a deuterium atom; a cyano group; a nitro group; a halogen atom, such as a fluorine atom, a chlorine atom, a bromine atom, and an iodine atom; a silyl group, such as a trimethylsilyl group and a triphenylsilyl group; a linear or branched alkyl group of 1 to 6 carbon atoms, such as a methyl group, an ethyl group, and a propyl group; a linear or branched alkyloxy group of 1 to 6 carbon atoms, such as a methyloxy group, an ethyloxy group, and a propyloxy group; an alkenyl group, such as a vinyl group and an allyl group; an aryloxy group, such as a phenyloxy group and a tolyloxy group; an arylalkyloxy group, such as a benzyloxy group and a phenethyloxy group; an aromatic hydrocarbon group or a condensed polycyclic aromatic group, such as a phenyl group, a biphenyl group, terphenyl group, a naphthyl group, an anthracenyl group, a phenanthrenyl group, an indenyl group, a pyrenyl group, a perylenyl group, a fluoranthenyl group, a triphenylenyl group; an aromatic heterocyclic group such as pyridyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl. These substituents may be further substituted with the exemplified substituents above. The substituent and the benzene ring substituted with the substituents or a plurality of the substituent on the same benzene ring may bind to each other via a single bond, substituted or unsubstituted methylene, an oxygen atom, or a sulfur atom to form a ring.

Specific examples of the "aromatic hydrocarbon", the "aromatic heterocyclic ring", or the "condensed polycyclic aromatics" in the "divalent group of an aromatic hydrocarbon", the "divalent group of an aromatic heterocyclic ring", or the "divalent group of condensed polycyclic aromatics" in the "substituted or unsubstituted divalent group of an aromatic hydrocarbon", the "substituted or unsubstituted divalent group of an aromatic heterocyclic ring", or the "substituted or unsubstituted divalent group of condensed polycyclic aromatics" represented by L in the general formulas (1) and (2) include benzene, biphenyl, terphenyl, naphthalene, anthracene, phenanthrene, indene, pyrene, perylene, fluoranthene, triphenylen, pyridine, pyrimidine, triazine, furan, pyrrole, thiophene, quinoline, isoquinoline, benzofuran, benzothiophene, indole, carbazole, benzoxazole, benzothiazole, azafluorene, diazafluorene, azaspirobifluorene, diazaspirobifluorene, quinoxaline, benzimidazole, pyrazole, dibenzofuran, dibenzothiophene, naphthyridine, phenanthroline, acridine, and carboline, and an aryl of 6 to 30 carbon atoms, or a heteroaryl of 2 to 20 carbon atoms may be selected. The "divalent group of an aromatic hydrocarbon", the "divalent group of an aromatic heterocyclic ring", or the "divalent group of condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by L in the general formulas (1) and (2) is a divalent group that results from the removal of two hydrogen atoms from the above "aromatic hydrocarbon", "aromatic heterocyclic ring", or "condensed polycyclic aromatics".

Examples of the "substituent" in the "divalent group of an aromatic hydrocarbon having a substituent", the "divalent group of an aromatic heterocyclic ring having a substituent", or the "divalent group of condensed polycyclic aromatics having a substituent" represented by L in the general formulas (1) and (2) include the same substituents exemplified as the "substituent" in the "substituted aromatic hydrocarbon group", the "substituted aromatic heterocyclic ring group", or the "substituted condensed polycyclic aromatic group", represented by Ar₁ and Ar₂ in the general formulas (1) and (2), and possible embodiments may also be the same embodiments as the exemplified embodiments.

Examples of the "substituent" in the "naphthyl group having a substituent", the "dibenzofuranyl group having a substituent", the "dibenzothienyl group having a substituent", the "carbazolyl group having a substituent", the "triphenylsilyl group having a substituent", or the "phenanthrenyl group having a substituent" represented by A in the general formulas (1) and (2) include the same substituents exemplified as the "substituent" in the "substituted aromatic hydrocarbon group", the "substituted aromatic heterocyclic ring group", or the "substituted condensed polycyclic aromatic group" represented by Ar₁ and Ar₂ in the general formulas (1) and (2), and possible embodiments may also be the same embodiments as the exemplified embodiments.

In the general formulas (1) and (2), one or both of Ar₁ and Ar₂ is preferably a substituted or unsubstituted phenyl group, a substituted or unsubstituted biphenyl group, a substituted or unsubstituted naphthyl group, a substituted or unsubstituted dibenzofuranyl group, a substituted or unsubstituted dibenzothienyl group, or a substituted or unsubstituted phenanthrenyl group, more preferably, a substituted or unsubstituted phenyl group, or a substituted or unsubstituted biphenyl group, further preferably, a substituted or unsubstituted phenyl group.

In the general formula (1) or (2), L is preferably a divalent group that results from the removal of two hydrogen atoms from unsubstituted benzene (phenylene group), a divalent group that results from the removal of two hydrogen atoms from unsubstituted biphenyl (biphenylene group), or a divalent group that results from the removal of two hydrogen atoms from unsubstituted naphthalene (naphthylene group), more preferably, a divalent group that results from the removal of two hydrogen atoms from unsubstituted benzene (phenylene group), or a divalent group that results from the removal of two hydrogen atoms from unsubstituted biphenyl (biphenylene group).

In the general formula (1) or (2), A is preferably an unsubstituted naphthyl group, an unsubstituted dibenzofuranyl group, an unsubstituted dibenzothienyl group, an unsubstituted carbazolyl group, an unsubstituted triphenylsilyl group, or an unsubstituted phenanthrenyl group, more preferably, an unsubstituted naphthyl group, an unsubstituted dibenzofuranyl group, an unsubstituted carbazolyl group, or an unsubstituted phenanthrenyl group, further preferably, an unsubstituted naphthyl group, an unsubstituted dibenzofuranyl group, or an unsubstituted phenanthrenyl group.

Here, a naphthyl group, a dibenzofuranyl group, a dibenzothienyl group, a carbazolyl group, or a phenanthrenyl group is preferably 1-naphthyl group, 2-naphthyl group, 1-dibenzofuranyl group, 2-dibenzofuranyl group, 3-a dibenzofuranyl group, 4-dibenzofuranyl group, 3-a dibenzothienyl group, 4-dibenzothienyl group, 9-carbazolyl group, or 9-phenanthrenyl group.

The arylamine compound of the general formula (1) or (2) preferably used in the organic EL devices of the present invention is preferably used as a constitutive material of a hole injection layer, a hole transport layer, an electron blocking layer, or a light emitting layer in the organic EL devices, more preferably, a constitutive material of a hole transport layer, or an electron blocking layer.

The arylamine compound of the general formula (1) or (2) of the present invention is preferably used as a constitutive material of the organic layer in the electronic apparatus having a pair of electrodes and at least one organic layer sandwiched therebetween.

### Effects of the Invention

The arylamine compound of the general formula (1) or (2) of the present invention has the following characteristics, than conventional hole transport materials, such as (1) better hole injection characteristics, (2) greater hole mobility, (3) more excellent electron blocking ability, (4) higher electron resistance, (5) more stable thin-film state, (6) more excellent heat resistance. Using the arylamine compound of the general formula (1) or (2) of the present invention for an organic EL device makes it possible to obtain characteristics, such as (1) high luminous efficiency, (2) low turn on voltage, (3) low actual driving voltage, (4) a long lifetime.

The arylamine compound of the general formula (1) or (2) of the present invention is excellent in hole injection and transport abilities, thin film stability, and durability. Thereby, the organic EL device having a hole injection layer and/or a hole transport layer fabricated using the compound as a hole injection material and/or a hole transport material can improve hole transport efficiency to the light emitting layer, luminous efficiency, and lower driving voltage. Thus, the durability of the device can be improved, and the characteristics of high efficiency, low driving voltage, and long lifetime can be obtained.

The arylamine compound of the general formula (1) or (2) of the present invention has the characteristics, such as excellent electron blocking ability, high electron resistance, thin film stability, and confining excitons generated in a light emitting layer. The organic EL device having an electron blocking layer fabricated using the compound as electron blocking material improves the probability of hole-electron recombination, and suppresses thermal deactivation, therefore, has high luminous efficiency. The driving voltage lowers and the current resistance improves, as a result, the maximum emission luminance improves.

The arylamine compound of the general formula (1) or (2) of the present invention has excellent hole transportability and a wide band gap. Therefore, the organic EL device having a light emitting layer fabricated using the compound as a host material forms the light emitting layer by carrying a fluorescent-emitting material, a phosphorescent-emitting material, or a delayed fluorescent-emitting material called a dopant, in this way, the driving voltage is lowered and the luminous efficiency is improved.

Thus, the arylamine compound of the general formula (1) or (2) of the present invention is useful as a constituent material of a hole injection layer, a hole transport layer, an electron blocking layer, or a light emitting layer of the organic EL device, and can improve luminous efficiency, driving voltage and durability of the conventional organic EL devices.

In addition, the arylamine compound of the general formula (1) or (2) of the present invention can be used not only in an organic EL device, but also in the field of electronic apparatus such as an electrophotographic photoreceptor, an image sensor, a photoelectric conversion device, and a solar cell.

### Brief Description of the Drawings

FIG. 1 is a figure showing the structures of Compounds (1) to (15) as an arylamine compound represented by the general formula (1) or (2).
FIG. 2 is a figure showing the structures of Compounds (16) to (30) as the arylamine compound represented by the general formula (1) or (2).
FIG. 3 is a figure showing the structures of Compounds (31 to (45) as the arylamine compound represented by the general formula (1) or (2).
FIG. 4 is a figure showing the structures of Compounds (46) to (60) as the arylamine compound represented by the general formula (1) or (2).
FIG. 5 is a figure showing the structures of Compounds (61) to (75) as the arylamine compound represented by the general formula (1) or (2).
FIG. 6 is a figure showing the structures of Compounds (76) to (90) as the arylamine compound represented by the general formula (1) or (2).
FIG. 7 is a figure showing the structures of Compounds (91) to (102) as the arylamine compound represented by the general formula (1) or (2).
FIG. 8 is a figure showing the structures of Compounds (103) to (117) as the arylamine compound represented by the general formula (1) or (2).
FIG. 9 is a figure showing the structures of Compounds (118) to (132) as the arylamine compound represented by the general formula (1) or (2).
FIG. 10 is a figure showing the structures of Compounds (133) to (145) as the arylamine compound represented by the general formula (1) or (2).
FIG. 11 is a figure showing the structures of Compounds (146) to (160) as the arylamine compound represented by the general formula (1) or (2).
FIG. 12 is a figure showing the structures of Compounds (161) to (162) as the arylamine compound represented by the general formula (1) or (2).
FIG. 13 is a diagram illustrating the configuration of the EL devices of Examples 31 to 58 and Comparative Examples 1 to **2.**

### Description of Embodiments

The arylamine compounds of the general formula (1) or (2) of the present invention are novel compounds, but these compounds per se can be synthesized according to a known method.

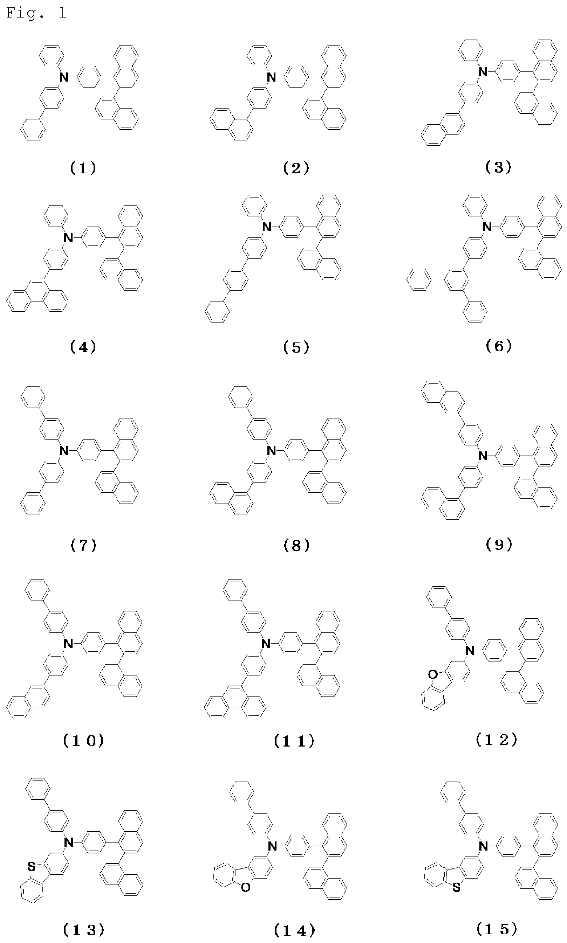

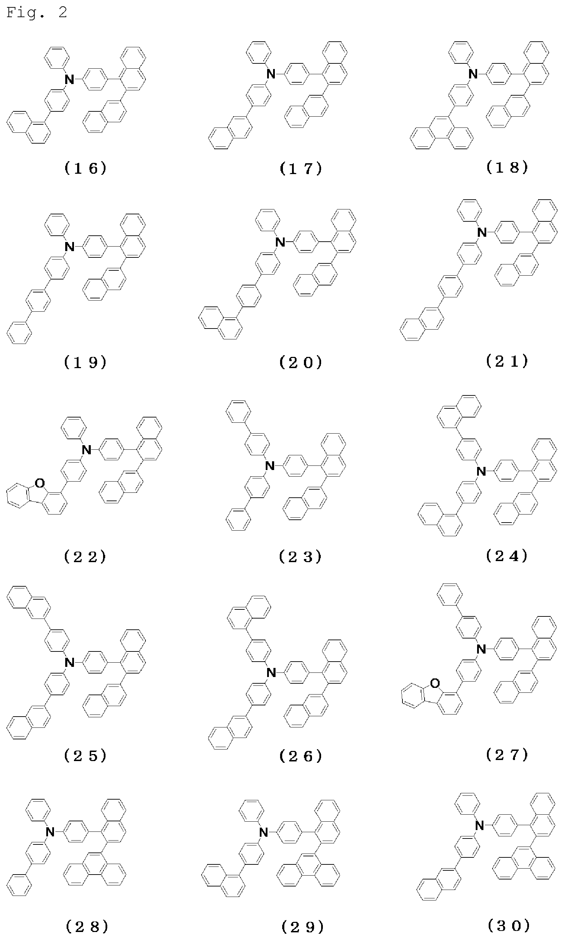

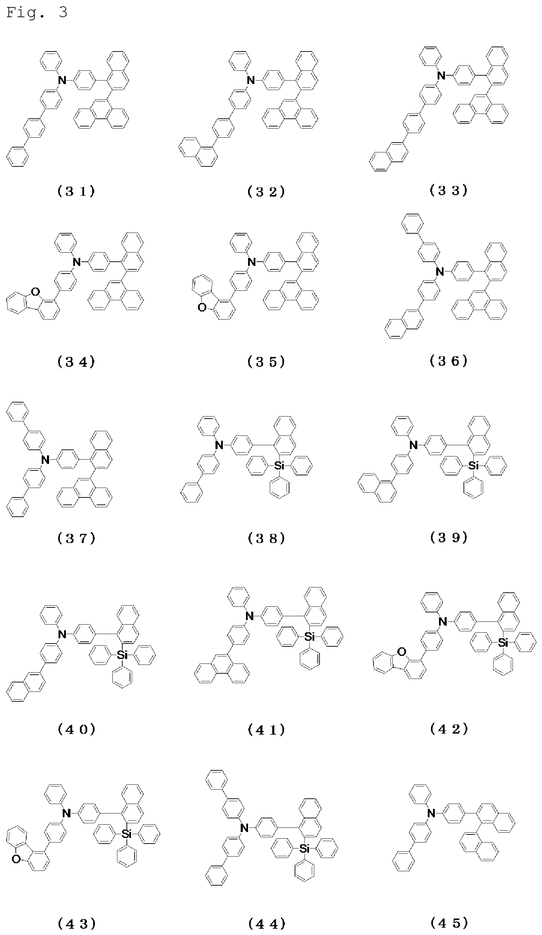

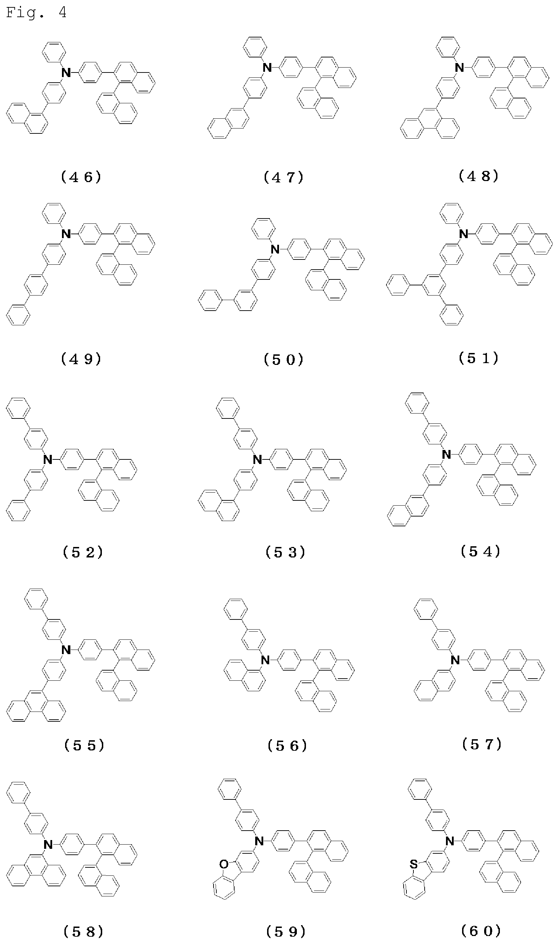

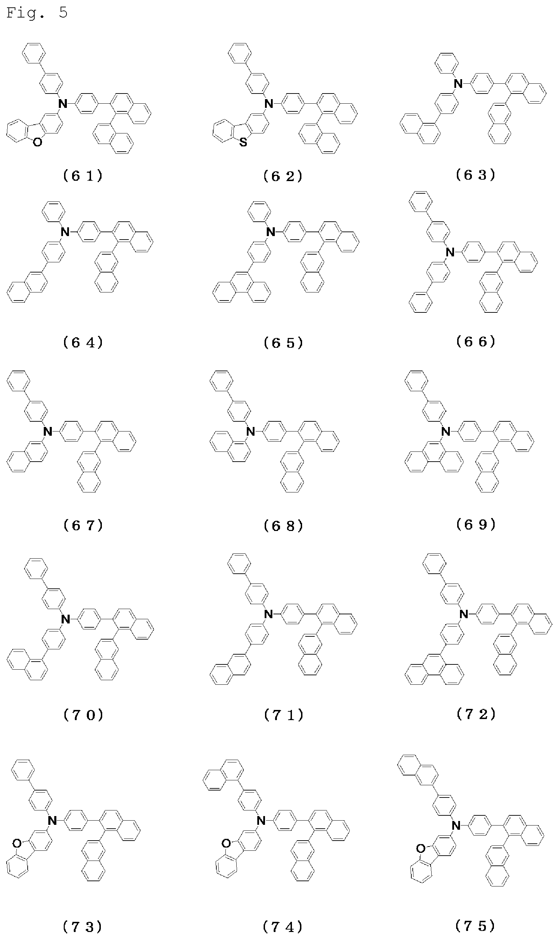

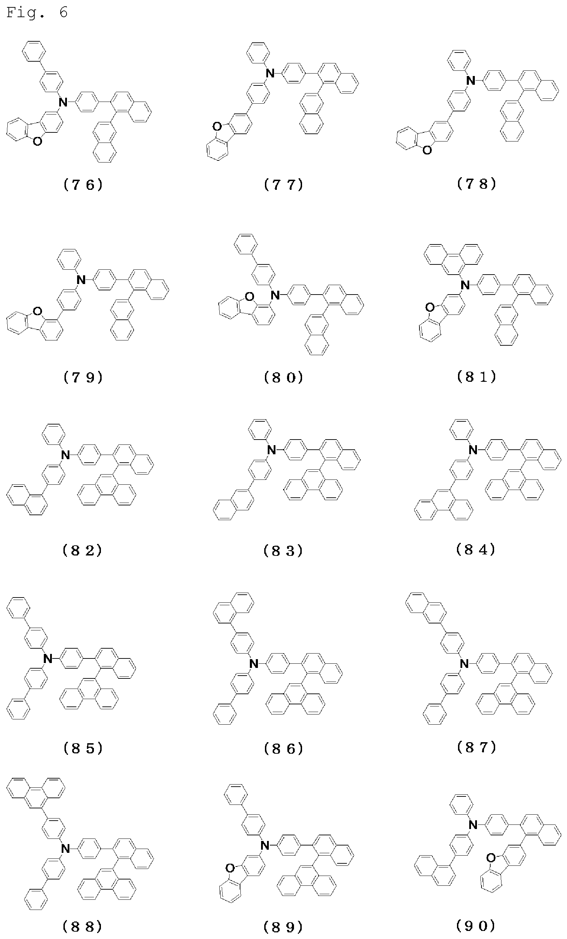

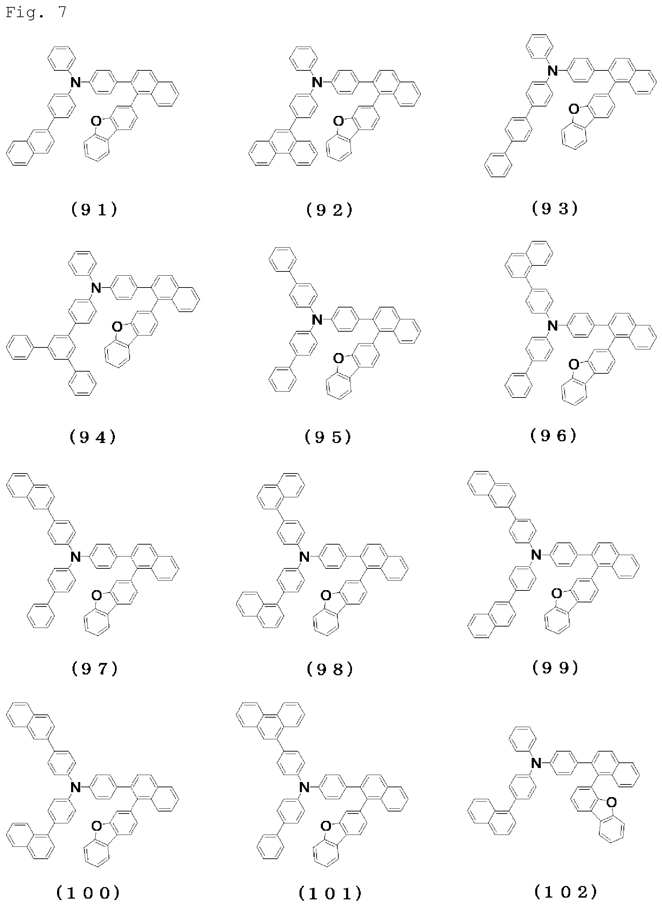

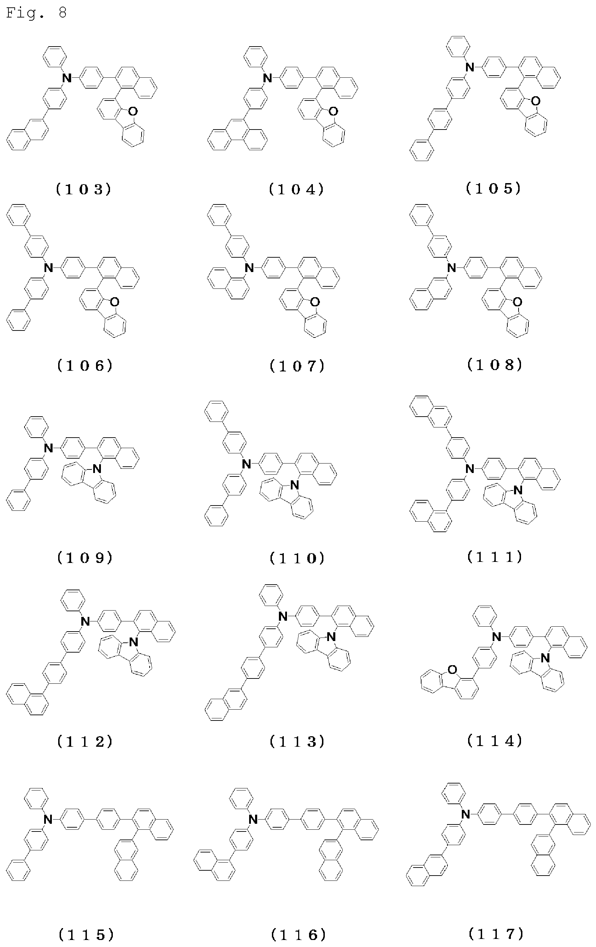

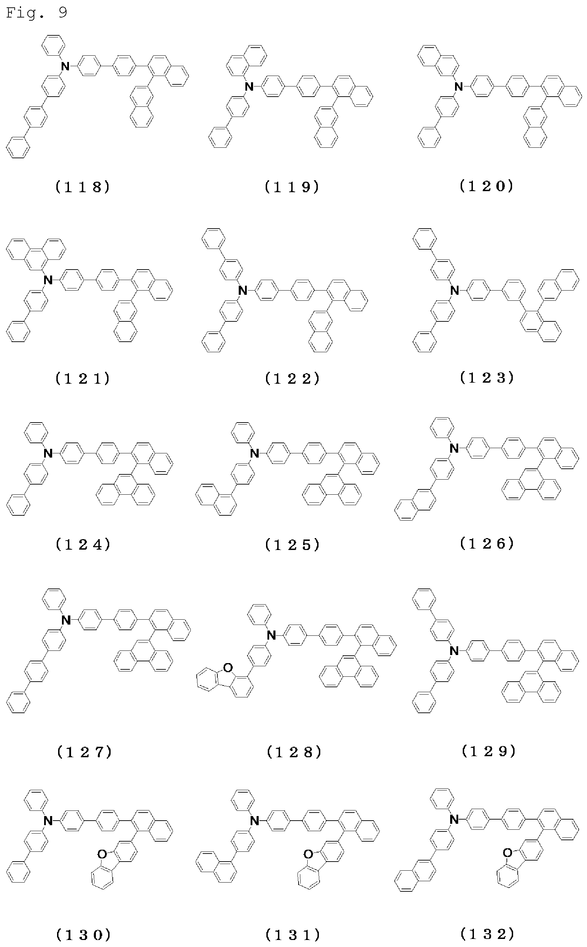

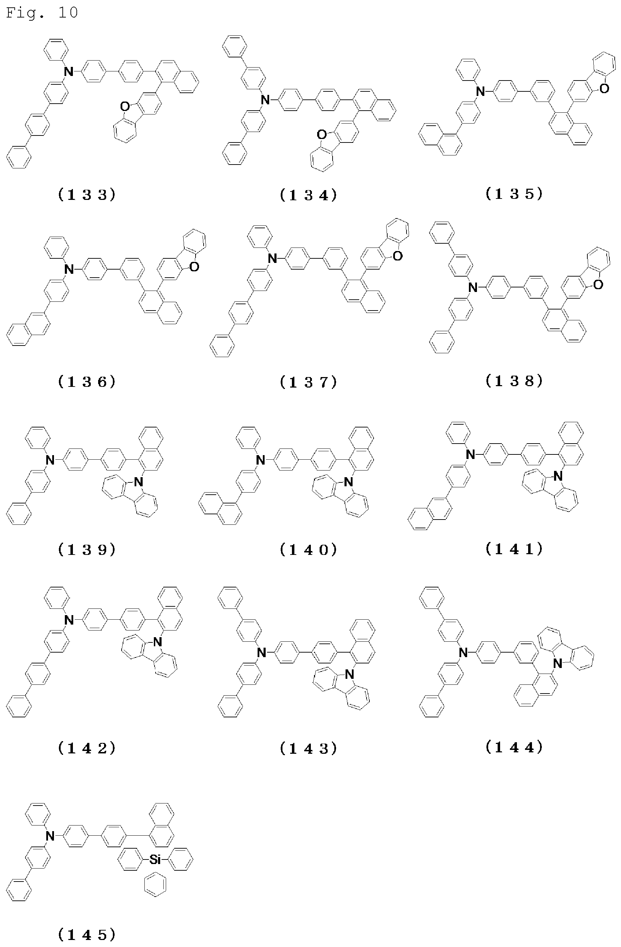

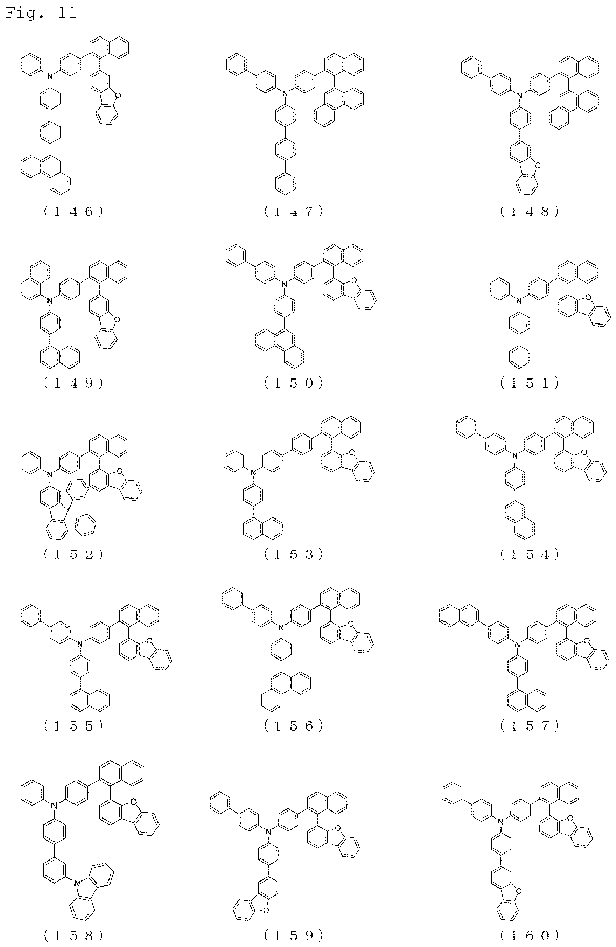

The specific examples of preferred compounds of the arylamine compound of the general formula (1) or (2) preferably used in the organic EL devices of the present invention are shown in Figs 1 to 12. The present invention, however, is not restricted to these compounds.

The purification of the arylamine compound of the general formula (1) or (2) of the present invention can be performed by a known method such as column chromatography, adsorption purification using, for example, a silica gel, activated carbon, or activated white clay, recrystallization or crystallization using a solvent, sublimation purification method, and finally, the purification was performed by the sublimation purification method.

The compounds were identified by an NMR analysis. A melting point, a glass transition point (Tg), and a work function were measured as material property values. The melting point can be used as an index of the deposition properties, the glass transition point (Tg) can be used as an index of the stability in a thin film state, and the work function can be used as an index of hole injectability, hole transportability, or electron blocking performance.

Other compounds used for the organic EL device of the present invention were purified by methods such as column chromatography, adsorption using, for example, a silica gel, activated carbon, or activated clay, and recrystallization or crystallization using a solvent, and finally purified by a sublimation purification method.

The melting point and the glass transition point (Tg) may be measured by, for example, a high-sensitive differential scanning calorimeter (DSC3100SA produced by Bruker AXS) using powder.

For the measurement of the work function, for example, a 100 nm thin film was fabricated on an ITO substrate, and an ionization potential measuring device (PYS-202 produced by Sumitomo Heavy Industries, Ltd.) may be used.

The organic EL device of the present invention may have a structure including an anode, a hole injection layer, a hole transport layer, a light emitting layer, an electron transport layer, an electron injection layer, and a cathode successively formed on a substrate, optionally with an electron blocking layer between the hole transport layer and the light emitting layer, and a hole blocking layer between the light emitting layer and the electron transport layer.

A single organic layer in the multilayer structure may serve as several layers. For example, a single organic layer may serve as the hole injection layer and the hole transport layer, or as the electron injection layer and the electron transport layer. Further, any of the layers may be configured to laminate two or more organic layers having the same function, and the hole transport layer may have a two-layer laminated structure, the light emitting layer may have a two-layer laminated structure, the electron transport layer may have a two-layer laminated structure.

Electrode materials with high work functions such as ITO and gold are used as the anode of the organic EL device of the present invention.

As the material of the hole injection layer of the organic EL device of the present invention, it is preferable to use the arylamine compound having only one triphenylamine structure within a molecule such as the arylamine compound of the general formula (1) or (2) of the present invention. Further, a porphyrin compound as represented by copper phthalocyanine; a starburst-type triphenylamine derivative; an arylamine compound having a structure in which two or more triphenylamine structures or carbazolyl structures within a molecule are joined via a single bond or a divalent group that does not contain a heteroatom; an accepting heterocyclic compound, such as hexacyanoazatriphenylene; and a coating-type polymer material may be used. These materials may be formed into a thin film by known methods such as a vapor deposition method, a spin coating method, and an inkjet method.

As the material of the hole injection layer and the hole transport layer of the organic EL device of the present invention, it is preferable to use an arylamine compound having only one triphenylamine structure within a molecule such as the arylamine compound of the general formula (1) or (2) of the present invention. Further, a benzidine derivative such as N,N'-diphenyl-N,N'-di(m-tolyl)-benzidine (TPD), N,N'-diphenyl-N,N'-di(α-naphthyl)-benzidine (NPD), and N,N,N',N'-tetrabiphenylylbenzidine; 1,1-bis[(di-4-tolylamino)phenyl]cyclohexane (TAPC); and an arylamine compound having a structure in which two or more triphenylamine structures or carbazolyl structures within a molecule are joined via a single bond or a divalent group that does not contain a heteroatom may be used. These materials may be individually deposited for film forming, the mixed several materials may be used for film forming, and each may be used as a single layer. These materials may be formed a laminate structure of individually deposited layers, a laminate structure of the deposited layers with mixed several materials, or a laminate structure of the individually deposited layer and the deposited layer with mixed several materials. A coating-type polymer material, such as poly(3,4-ethylenedioxythiophene) (PEDOT)/poly (styrene sulfonate) (PSS) may be used as the material of the injection and transport layer of the hole. These materials may be formed into a thin film by known methods such as a vapor deposition method, a spin coating method, and an inkjet method.

The material used for the hole injection layer or the hole transport layer may be obtained by p-doping material such as trisbromophenylaminehexachloroantimony, and a radialene derivative (refer to Patent Document 6, for example) into a material commonly used for these layers. Further, polymer compounds each having, as a part of the compound structure, a structure of a benzidine derivative such as TPD may be used.

As the material of the electron blocking layer of the organic EL device of the present invention, it is preferable to use the arylamine compound of the general formula (1) or (2) of the present invention. Further, a compound having an electron blocking effect such as a carbazole derivative, such as 4,4',4' '-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz); and a compound having a triphenylsilyl group and a triarylamine structure, as represented by 9-[4-(carbazol-9-yl)phenyl]-9-[4-(triphenylsilyl)phenyl]-9H-fluorene may be used. These materials may serve as the material of the hole transport layer. These materials may be individually deposited for film forming, the mixed several materials may be used for film forming, and each may be used as a single layer. These materials may be formed a laminate structure of individually deposited layers, a laminate structure of the deposited layers with mixed several materials, or a laminate structure of the individually deposited layer and the deposited layer with mixed several materials. These materials may be formed into a thin film by known methods such as a vapor deposition method, a spin coating method, and an inkjet method.

As the material used for the light emitting layer of the organic EL device of the present invention, it is preferable to use the arylamine compound of the general formula (1) or (2) of the present invention. Further, metal complexes of a quinolinol derivative such as tris(8-quinolinolato)aluminum (Alq₃); various metal complexes; an anthracene derivative; a bis-styrylbenzene derivative; a pyrene derivative; an oxazole derivative; and a polyparaphenylene vinylene derivative can be used. Further, the light emitting layer may be made of a host material and a dopant material. In that case, as the host material, the arylamine compound of the general formula (1) or (2) of the present invention, or an anthracene derivative is preferably used. In addition to these materials, it may be also to use a heterocyclic compound having an indole ring as a partial structure of the fused ring; a heterocyclic compound having a carbazole ring as a partial structure of fused ring; a carbazole derivative; a thiazole derivative; a benzimidazole derivative; and a polydialkylfluorene derivative. Further, as the dopant material, a heterocyclic compound having S, B, N, or the like as a ring-constituting element may be used. Furthermore, quinacridone, coumarin, rubrene, perylene, and derivatives thereof; a benzopyran derivative; a rhodamine derivative; and an aminostyryl derivative can be used. These materials may be individually deposited for film forming, the mixed several materials may be used for film forming, and each may be used as a single layer. These materials may be formed a laminate structure of individually deposited layers, a laminate structure of the deposited layers with mixed several materials, or a laminate structure of the individually deposited layer and the deposited layer with mixed several materials. These materials may be formed into a thin film by known methods such as a vapor deposition method, a spin coating method, and an inkjet method.

Further, a phosphorescent material may be used as the light emitting material. For example, metal complexes such as iridium and platinum may be used as phosphorescent materials. Examples of the phosphorescent materials include green phosphorescent materials such as Ir(ppy)₃, blue phosphorescent materials such as FIrpic and FIr6, and red phosphorescent materials such as Btp₂Ir(acac). For the host material, the arylamine compound of the general formula (1) or (2) of the present invention in addition to a carbazole derivative such as 4,4'-di(N-carbazolyl)biphenyl (CBP), TCTA, and mCP may be used as the hole injecting and transporting host material. As the electron transporting host material, a compound such as p-bis(triphenylsilyl)benzene (UGH2) and 2,2',2''-(1,3,5-phenylene)-tris(1-phenyl-1H-benzimidazole) (TPBI) may be used. A high-performance organic EL device can be fabricated by such materials.

In order to avoid concentration quenching, the doping of the host material with the phosphorescent light-emitting material should preferably be made by co-evaporation in a range of 1 to 30 weight percent with respect to the whole light emitting layer.

Further, as the light-emitting material, delayed fluorescent-emitting material such as a CDCB derivative of PIC-TRZ, CC2TA, PXZ-TRZ, and 4CzIPN (refer to Non-Patent Document 3, for example) may be used. These materials may be formed into a thin film by known methods such as a vapor deposition method, a spin coating method, and an inkjet method.

As the material used for the hole blocking layer of the organic EL device of the present invention, a hole blocking compound, such as metal complexes of a phenanthroline derivative such as bathocuproin (BCP), and a quinolinol derivative such as bis(2-methyl-8-quinolinolate)-4-(phenylphenolato)aluminium (BAlq); various rare earth complexes; an oxazole derivative; a triazole derivative; and a triazine derivative. These materials may serve as the material of the electron transport layer. These materials may be individually deposited for film forming, the mixed several materials may be used for film forming, and each may be used as a single layer. These materials may be formed a laminate structure of individually deposited layers, a laminate structure of the deposited layers with mixed several materials, or a laminate structure of the individually deposited layer and the deposited layer with mixed several materials. These materials may be formed into a thin film by known methods such as a vapor deposition method, a spin coating method, and an inkjet method.

As the material used for the electron transport layer of the organic EL device of the present invention, it is preferable to use a benzimidazole derivative or an anthracene derivative. Further, metal complexes of a quinolinol derivative such as Alq₃ and BAlq, various metal complexes, a triazole derivative, a triazine derivative, an oxadiazole derivative, a pyridine derivative, a pyrimidine derivative, a thiadiazole derivative, a carbodiimide derivative, a quinoxaline derivative, a pyridoindole derivative, a phenanthroline derivative, and a silole derivative may be used. These materials may be individually deposited for film forming, the mixed several materials may be used for film forming, and each may be used as a single layer. These materials may be formed a laminate structure of individually deposited layers, a laminate structure of the deposited layers with mixed several materials, or a laminate structure of the individually deposited layer and the deposited layer with mixed several materials. These materials may be formed into a thin film by known methods such as a vapor deposition method, a spin coating method, and an inkjet method.

As the material of the electron injection layer of the organic EL device of the present invention, alkali metal salts such as lithium fluoride and cesium fluoride; alkaline earth metal salts such as magnesium fluoride; metal complexes of a quinolinol derivative such as lithium quinolinol; metal oxides such as aluminum oxide; and metals such as itterbium (Yb), samarium (Sm), calcium (Ca), strontium (Sr), and cesium (Cs). However, the electron injection layer may be omitted in the preferred selection of the electron transport layer and the cathode. The electron injection layer may be omitted in the preferred selection of the electron transport layer and the cathode.

Further, the material used for the electron injection layer or the electron transport layer may be obtained by N-doping metal such as cecium into a material commonly used for these layers.

As the cathode of the organic EL device of the present invention, metal with a low work function such as aluminum, or an alloy with an even lower work function such as a magnesium-silver alloy, a magnesium-indium alloy, and an aluminum-magnesium alloy may be used as an electrode material. Examples

The following describes an embodiment of the present invention in more detail based on examples. The present invention, however, is not restricted to the following examples as long as they do not go beyond the claims of the invention.

### Example 1

### <Synthesis of [4-{1-(dibenzofuran-3-yl)naphthalene-2-yl}phenyl]-{4-(naphthalene-2-yl)phenyl}-phenylamine (Compound (91))>

1-bromo-2-naphthol: 20.0g, 3-dibenzofuranboronicacid: 22.8g, tetrakis (triphenylphosphine)palladium(0): 1.0g, and potassium carbonate: 18.6g were added into a reaction vessel, the mixture was refluxed and stirred in a THF/water mixed solvent for 15 hours. After confirming the completion of the reaction, the mixture was allowed to cool, and added a toluene/water mixed solvent. An organic layer was collected by extraction and liquid separation, and then concentrated to obtain a crude product. The obtained crude product was purified by recrystallizing with an n-heptane solvent, whereby 1-(dibenzofuran-3-yl)-2-naphthol: 16.4 g (yield: 59.0%) was obtained.

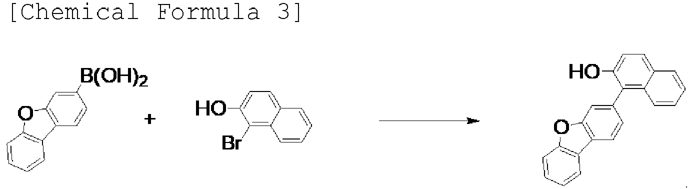

Then, the obtained 1-(dibenzofuran-3-yl)-2-naphthol: 9.0g, and pyridine: 3.4g were added into a reaction vessel. After stirring in a dichloromethane solvent in ice-cold, trifluoromethanesulfonic anhydride: 9.0g was added thereto dropwise, and the mixture was stirred at room temperature overnight. After confirming the completion of the reaction, water was added thereto, an organic layer was collected by extraction and liquid separation, and then concentrated to obtain a crude product. The obtained crude product was purified using column chromatography (support: silica gel, eluent: a dichloromethane/n-heptane mixed solvent), whereby 1-(dibenzofuran-3-yl)naphthalene-2-yl-trifluoromethanesulfonate: 9.3g (yield: 73.0%) was obtained.

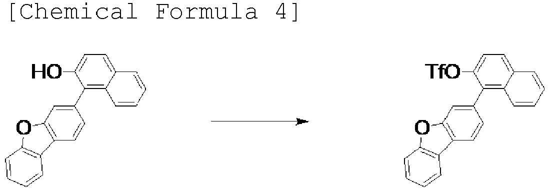

Then, 1-(dibenzofuran-3-yl)naphthalene-2-yl-trifluoromethanesulfonate obtained by repeating: 45.0g, {4-(4,4,5,5-tetramethyl-[1,3,2]dioxaborolane-2-yl)phenyl}-phenylamine: 31.5g, tetrakis(triphenylphosphine)palladium(0): 2.4g, and potassium carbonate: 281g were added into a reaction vessel, the mixture was refluxed and stirred in a toluene/ethanol/water mixed solvent overnight. After confirming the completion of the reaction, a toluene/water mixed solvent was added thereto. An organic layer was collected by extraction and liquid separation, and then concentrated to obtain a crude product. The obtained crude product was purified by crystallization with a dichloromethane/n-heptane mixed solvent, whereby [4-{1-(dibenzofuran-3-yl)naphthalene-2-yl}phenyl]-phenylamine: 42.4g (yield: 90.4%) was obtained.

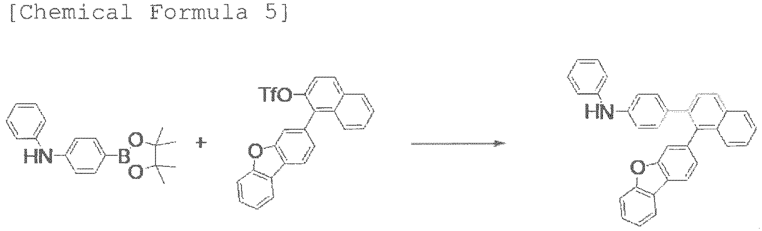

Then, the obtained [4-{1-(dibenzofuran-3-yl)naphthalene-2-yl}phenyl]-phenylamine: 14.0g, 2-(4-bromophenyl) naphthalene: 9.4g, palladium acetate(II): 0.1g, tri-tert-butylphosphine: 0.3g, and tert-butoxy sodium: 3.5g were added into a reaction vessel, the mixture was refluxed and stirred in a toluene solvent overnight. After confirming the completion of the reaction, the filtrate obtained by filtration was concentrated to obtain a crude product. The obtained crude product was purified by crystallization with a toluene/acetone mixed solvent, whereby a white powder of [4-{1-(dibenzofuran-3-yl)naphthalene-2-yl}phenyl]-{4-(naphthalene-2-yl)phenyl}-phenylamine (compound (91)): 9.5g (yield: 47.2%) was obtained.

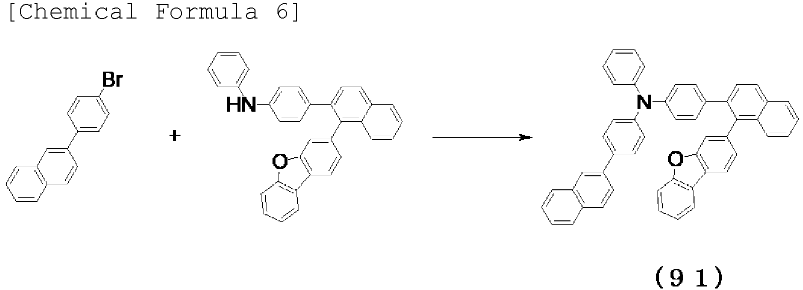

The structure of the obtained white powder was identified by NMR.

The ¹H-NMR(CDCl₃) detected 33 hydrogen signals, as follows.

δ (ppm) = 8.02 (1H), 7.98 (1H), 7.95 (1H), 7.94 (1H), 7.90 (1H), 7.86 (3H), 7.77 (1H), 7.67 (1H), 7.62(1H), 7.60 (1H), 7.49 (4H), 7.45-7.35 (5H), 7.29 (1H), 7.16 (2H), 7.04 (4H), 6.99 (2H), 6.97 (1H), 6.89 (2H).

### Example 2

### <Synthesis of [4-{1-(dibenzofuran-3-yl)naphthalene-2-yl}phenyl]-([1,1';4',1"]terphenyl-4-yl)-phenylamine (Compound (93))>

[4-{1-(dibenzofuran-3-yl)naphthalene-2-yl}phenyl]-phenylamine obtained in Example 1: 14.0g, 4-bromo-[1,1':4',1"]terphenyl: 10.3g, palladium acetate(II): 0.1g, tri-tert-butylphosphine: 0.3g, and tert-butoxy sodium: 3.5g were added into a reaction vessel, the mixture was refluxed and stirred in a toluene solvent overnight. After confirming the completion of the reaction, the filtrate obtained by filtration was concentrated to obtain a crude product. The obtained crude product was purified by crystallization with a toluene/acetone mixed solvent, whereby a white powder of [4-{1-(dibenzofuran-3-yl)naphthalene-2-yl}phenyl]-([1,1';4',1"]terphenyl-4-yl)-phenylamine (compound (93)): 9.6g (yield: 46.1%) was obtained.

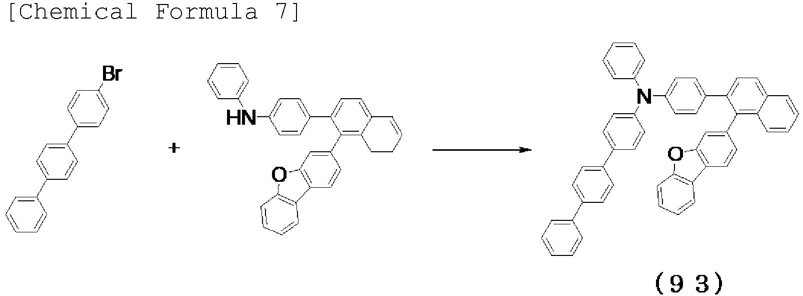

The structure of the obtained white powder was identified by NMR.

The ¹H-NMR(CDCl₃) detected 35 hydrogen signals, as follows.

δ (ppm) = 8.02 (1H), 7.97 (1H), 7.95 (1H), 7.94 (1H), 7.77 (1H), 7.70-7.59 (6H), 7.55-7.33 (10H), 7.31 (2H), 7.28 (1H), 7.15 (2H), 7.03 (4H), 6.97 (3H), 6.88 (2H).

### Example 3

### <Synthesis of (biphenyl-4-yl)-[4-{1-(dibenzofuran-3-yl)naphthalene-2-yl}phenyl]-(4-naphthalene-2-yl-phenyl)amine (Compound (97))>

1-(dibenzofuran-3-yl)naphthalene-2-yl-trifluoromethanesulfonate obtained in Example 1: 9.3g, (biphenyl-4-yl)-{4-(4,4,5,5-tetramethyl-[1,3,2]dioxaborolane-2-yl)phenyl}amine: 8.6g, tetrakis (triphenylphosphine)palladium(0): 0.2g, and potassium carbonate: 4.4g were added into a reaction vessel, the mixture was refluxed and stirred in a toluene/ethanol/water mixed solvent for 17 hours. After confirming the completion of the reaction, the mixture was allowed to cool, and added a methanol/water mixed solvent. A solid precipitated was collected by filtration to obtain a crude product. The obtained crude product was purified by crystallization with a chlorobenzene/acetone mixed solvent, whereby (biphenyl-4-yl)-[4-{1-(dibenzofuran-3-yl)naphthalene-2-yl}phenyl]amine: 7.2g (yield: 56.0%) was obtained.

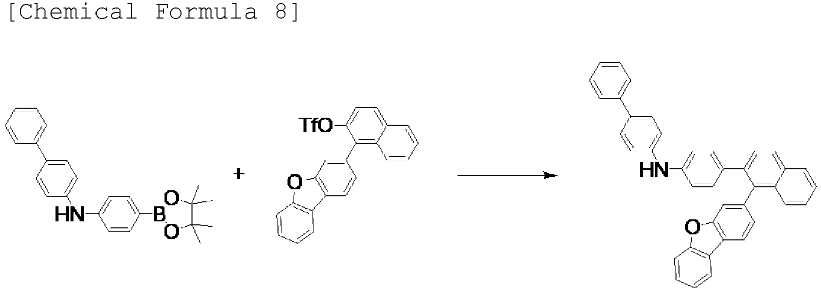

Then, the obtained (biphenyl-4-yl)-[4-{1-(dibenzofuran-3-yl)naphthalene-2-yl}phenyl]amine: 7.2g, 2-(4-bromophenyl)naphthalene: 4.2g, dipalladium acetate(II): 0.1g, tri-tert-butylphosphine: 0.1g, and tert-butoxy sodium: 1.9g were added into a reaction vessel, the mixture was refluxed and stirred in a toluene solvent for 3 hours. After allowing it to cool, the filtrate obtained by filtration was concentrated to obtain a crude product. The obtained crude product was purified by crystallization with a chlorobenzene/acetone mixed solvent, whereby a white powder of (biphenyl-4-yl)-[4-{1-(dibenzofuran-3-yl)naphthalene-2-yl}phenyl]-(4-naphthalene-2-yl-phenyl)amine (compound (97)): 5.1g (yield: 52.0%) was obtained.

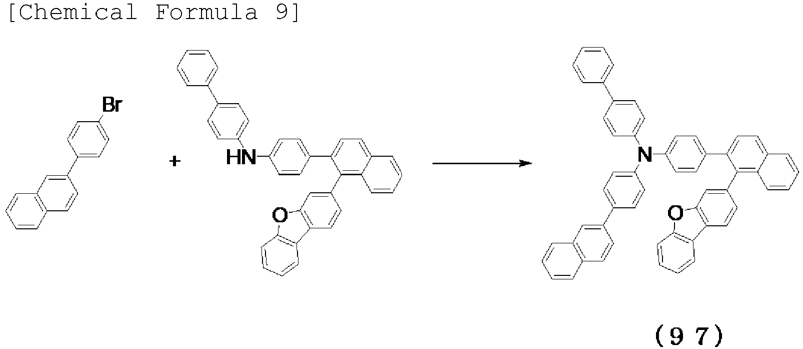

The structure of the obtained white powder was identified by NMR.

The ¹H-NMR(CDCl₃) detected 37 hydrogen signals, as follows.

δ (ppm) = 8.03 (1H), 7.99 (1H), 7.96 (2H), 7.92 (1H), 7.88 (2H), 7.85 (1H), 7.79 (1H), 7.69 (1H), 7.63 (2H), 7.55-7.36 (13H), 7.32 (4H), 7.06 (6H), 6.94 (2H).

### Example 4

### <Synthesis of [4'-{1-(dibenzofuran-3-yl)naphthalene-2-yl}biphenyl-4-yl]-(4-naphthalene-2-yl-phenyl)-phenylamine(Compound (132))>

1-(dibenzofuran-3-yl)naphthalene-2-yl-trifluoromethanesulfonate obtained in Example 1: 14.0g, {4'-(4,4,5,5-tetramethyl-[1,3,2]dioxaborolane-2-yl)biphenyl-4-yl}-phenylamine: 12.3g, tetrakis(triphenylphosphine)palladium(0): 0.7g, and potassium carbonate: 8.8g were added into a reaction vessel, the mixture was refluxed and stirred in a toluene/ethanol/water mixed solvent overnight. After confirming the completion of the reaction, a toluene/water mixed solvent was added thereto. An organic layer was collected by extraction and liquid separation, and then concentrated to obtain a crude product. The obtained crude product was purified by crystallization with a dichloromethane/n-heptane mixed solvent, whereby [4'-{1-(dibenzofuran-3-yl)naphthalene-2-yl}biphenyl-4-yl]-phenylamine: 11.1g (yield: 65.1%) was obtained.

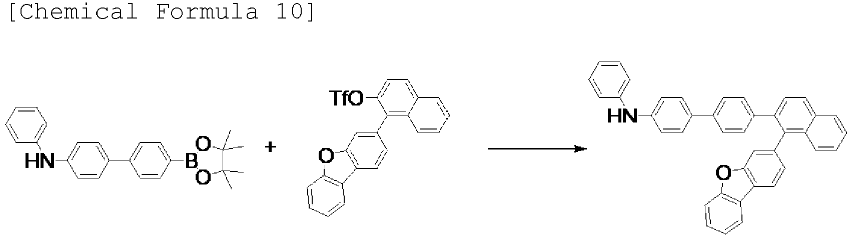

Then, the obtained [4'-{1-(dibenzofuran-3-yl)naphthalene-2-yl}biphenyl-4-yl]-phenylamine: 11.0g, 2-(4-bromophenyl)naphthalene: 6.4g, palladium acetate(II): 0.1g, tri-tert-butylphosphine: 0.2g, and tert-butoxy sodium: 2.4g were added into a reaction vessel, the mixture was refluxed and stirred in a toluene solvent overnight. After confirming the completion of the reaction, the filtrate obtained by filtration was concentrated to obtain a crude product. The obtained crude product was purified by recrystallizing with a chlorobenzene solvent, whereby a white powder of [4'-{1-(dibenzofuran-3-yl)naphthalene-2-yl}biphenyl-4-yl]-(4-naphthalene-2-yl-phenyl)-phenylamine (compound (132)): 8.7g (yield: 57.5%) was obtained.

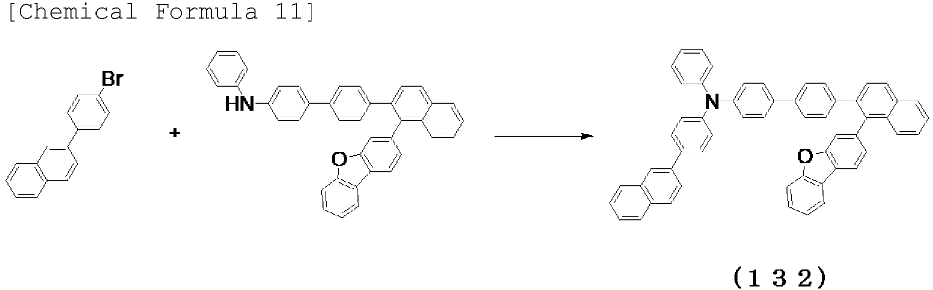

The structure of the obtained white powder was identified by NMR.

The ¹H-NMR(CDCl₃) detected 37 hydrogen signals, as follows.

δ (ppm) = 8.00 (1H), 7.98 (1H), 7.95 (2H), 7.89 (2H), 7.85 (2H), 7.70 (2H), 7.66 (1H), 7.59 (2H), 7.55 (1H), 7.52-7.31 (11H), 7.30-7.21 (5H), 7.18 (2H), 7.14 (4H), 7.04 (1H).

### Example 5

### <Synthesis of Compound (146)>

{4'-(phenanthrene-9-yl)biphenyl-4-yl}-phenylamine: 11.0g, 1-chloro-4-[{1-(dibenzofuran-3-yl)naphthalene-2-yl}phenyl]benzene: 11.6g, tert-butoxy sodium: 3.8g, tris(dibenzylideneacetone)palladium(0): 0.5g, and tri-tert-butylphosphine: 0.2g were added into a reaction vessel, the mixture was refluxed and stirred in a xylene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (146): 16.7g (yield: 81.0%)was obtained.

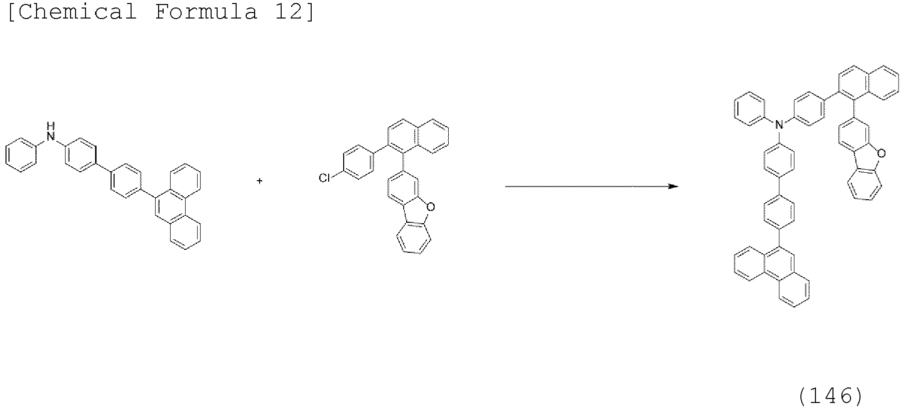

The structure of the obtained white powder was identified by NMR.

The ¹H-NMR (CDCl₃) detected 39 hydrogen signals, as follows.

δ (ppm) = 8.79 (1H), 8.73 (1H), 8.02-7.90 (6H), 7.78-7.56 (12H), 7.49 (2H), 7.41-7.35 (5H), 7.28 (1H), 7.16 (2H), 7.05 (4H), 7.01-6.96 (3H), 6.89 (2H).

### Example 6

### <Synthesis of Compound (103)>

{4-(naphthalene-2-yl)phenyl}-phenylamine: 7.5g, 1-chloro-4-{1-(dibenzofuran-4-yl)naphthalene-2-yl}benzene: 11.3g, tert-butoxy sodium: 3.7g, tris(dibenzylideneacetone)palladium(0): 0.5g, and tri-tert-butylphosphine: 0.2g were added into a reaction vessel, the mixture was refluxed and stirred in a xylene solvent overnight. After the reaction was completed, the insoluble matter was removed by filtration, then the resulting filtrate was concentrated to obtain a crude product. The obtained crude product was purified by crystallization with a toluene solvent, whereby a white powder of Compound (103): 14.2g (yield: 84.3%) was obtained.

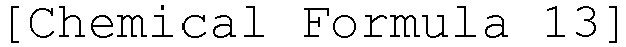

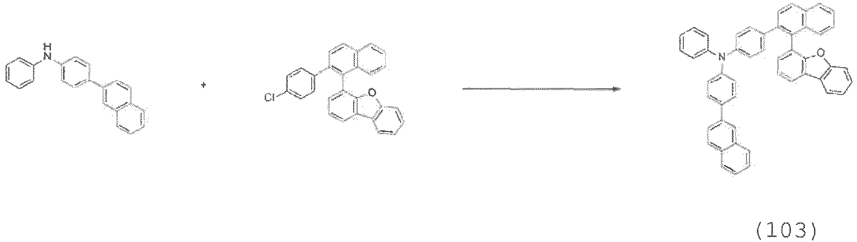

The structure of the obtained white powder was identified by NMR.

The ¹H-NMR (CDCl₃) detected 33 hydrogen signals, as follows.

δ (ppm) = 8.04 (1H), 8.01-7.96 (4H), 7.90-7.86 (3H), 7.74-7.68 (3H), 7.50-7.36 (11H), 7.13 (2H), 7.05 (2H), 6.95 (1H), 6.83 (4H), 6.78 (2H).

### Example 7

### <Synthesis of Compound (85)>

(biphenyl-4-yl)-4-{1-(phenanthrene-9-yl)naphthalene-2-yl}phenylamine: 13.0g, 4-bromobiphenyl: 6.1g, tert-butoxy sodium: 2.7g, tris(dibenzylideneacetone)palladium(0): 0.4g, and tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (85): 10.1g (yield: 60.8%) was obtained.

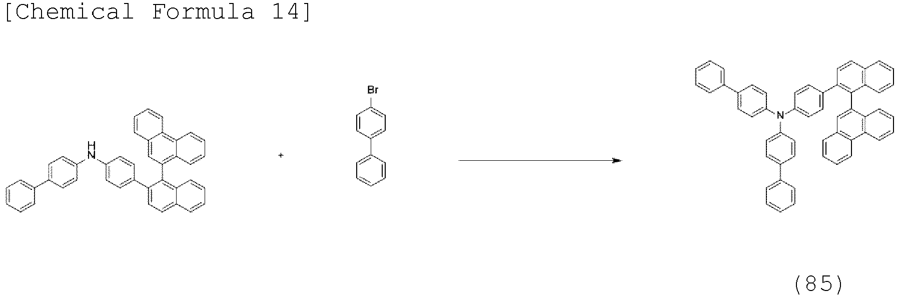

The structure of the obtained white powder was identified by NMR.

The ¹H-NMR (CDCl₃) detected 37 hydrogen signals, as follows.

δ (ppm) = 8.75 (2H), 8.04 (1H), 7.96 (1H), 7.79-7.74 (2H), 7.70 (1H), 7.61 (2H), 7.55-7.27 (20H), 7.02 (2H), 6.86 (4H), 6.72 (2H).

### Example 8

### <Synthesis of Compound (147)>

(biphenyl-4-yl)-4-{1-(phenanthrene-9-yl)naphthalene-2-yl}phenylamine: 13.0g, 4-bromo-[1,1' :4',1"]terphenyl: 8.1g, tert-butoxy sodium: 2.7g, tris(dibenzylideneacetone)palladium(0): 0.4g, tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a monochlorobenzene/acetone mixed solvent, whereby a white powder of Compound(147): 17.6g (yield: 95.6%) was obtained.

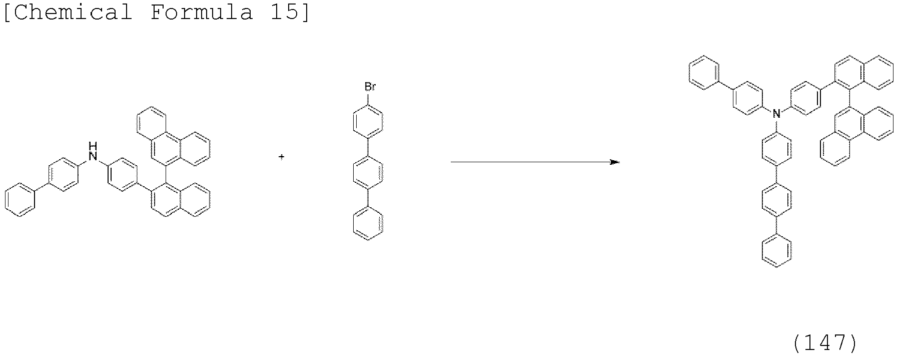

The structure of the obtained white powder was identified by NMR.

The ¹H-NMR (CDCl₃) detected 41 hydrogen signals, as follows.

δ (ppm) = 8.75 (2H), 8.04 (1H), 7.96 (1H), 7.79-7.70 (3H), 7.63-7.50 (11H), 7.46-7.23 (15H), 7.02 (2H), 6.86 (4H), 6.74 (2H).

### Example 9

### <Synthesis of Compound (148)>

(biphenyl-4-yl)-4-{1-(phenanthrene-9-yl)naphthalene-2-yl}phenylamine: 13.0g, 1-chloro-4-(dibenzofuran-3-yl)benzene: 7.3g, tert-butoxy sodium: 2.7g, tris(dibenzylideneacetone)palladium(0): 0.4g, tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified using column chromatography (support: silica gel, eluent: a dichloromethane/heptane mixed solvent), whereby a white powder of Compound (148): 8.3g (yield: 44.3%) was obtained.

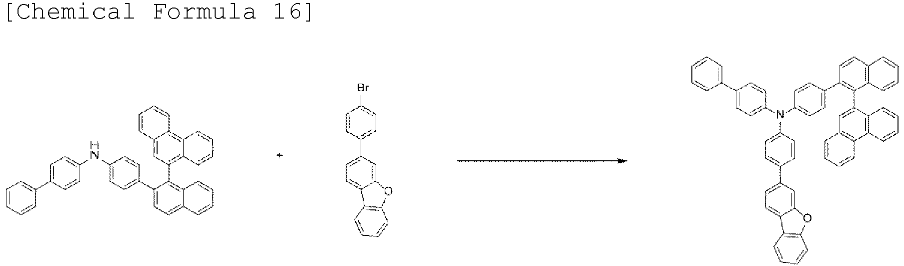

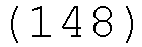

The structure of the obtained white powder was identified by NMR.

The ¹H-NMR (CDCl₃) detected 39 hydrogen signals, as follows.

δ (ppm) = 8.76 (2H), 8.04 (1H), 7.96-7.93 (3H), 7.78-7.70 (4H), 7.62-7.24 (21H), 7.03 (2H), 6.87 (4H), 6.74 (2H).

### Example 10

### <Synthesis of Compound (95)>

Bis(biphenyl-4-yl)-{4-(4,4,5,5-tetramethyl-1,3,2-dioxaborolan-2-yl)phenyl}amine: 12.0g, 1-(dibenzofuran-3-yl)naphthalene-2-yl-trifluoromethanesulfonate: 15.6g, potassium carbonate: 7.5g, tetrakistriphenylphosphine palladium(0): 0.6g were added into a reaction vessel, the mixture was refluxed and stirred in a toluene/ethanol/water mixed solvent overnight. After the reaction was completed, methanol was added thereto, and dispersion washing was carried out to obtain a crude product. The obtained crude product was purified by crystallization with a monochlorobenzene/acetone mixed solvent, whereby a white powder of Compound (95): 13.8g (yield: 73.7%) was obtained.

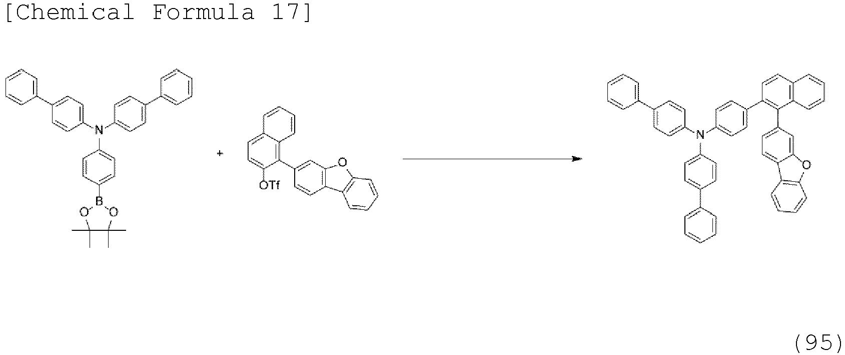

The structure of the obtained white powder was identified by NMR.

The ¹H-NMR (CDCl₃) detected 35 hydrogen signals, as follows.

δ (ppm) = 8.02-7.96 (4H), 7.80 (1H), 7.71 (1H), 7.64 (1H), 7.55-7.41 (13H), 7.35-7.28 (7H), 7.09-7.04 (6H), 6.94 (2H).

### Example 11

### <Synthesis of Compound (149)>

(naphthalene-1-yl)-{4-(naphthalene-1-yl)phenyl}amine: 9.0g, 1-chloro-4-{1-(dibenzofuran-3-yl)naphthalene-2-yl}benzene: 11.6g, tert-butoxy sodium: 3.8g, tris(dibenzylideneacetone)palladium(0): 0.5g, and tri-tert-butylphosphine: 0.2g were added into a reaction vessel, the mixture was refluxed and stirred in a xylene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a pale yellow powder of Compound (149): 10.9g (yield: 58.6%) was obtained.

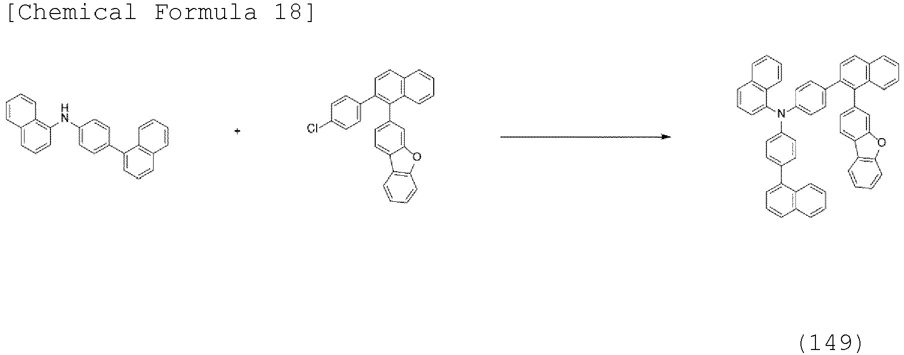

The structure of the obtained pale yellow powder was identified by NMR.

The ¹H-NMR (CDCl₃) detected 35 hydrogen signals, as follows.

δ (ppm) = 7.99-7.84 (9H), 7.75 (2H), 7.67-7.60 (2H), 7.51-7.36 (12H), 7.28-7.20 (4H), 7.05-7.02 (4H), 6.90 (2H).

### Example 12

### <Synthesis of Compound (100)>

{4-(naphthalene-1-yl)phenyl}-{4-(naphthalene-2-yl)phenyl}amine: 11.0g, 1-chloro-4-{1-(dibenzofuran-3-yl)naphthalene-2-yl}benzene: 11.6g, tert-butoxy sodium: 3.8g, tris(dibenzylideneacetone)palladium(0): 0.5g, and tri-tert-butylphosphine: 0.2g were added into a reaction vessel, the mixture was refluxed and stirred in a xylene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (100): 17.4g (yield: 84.4%) was obtained.

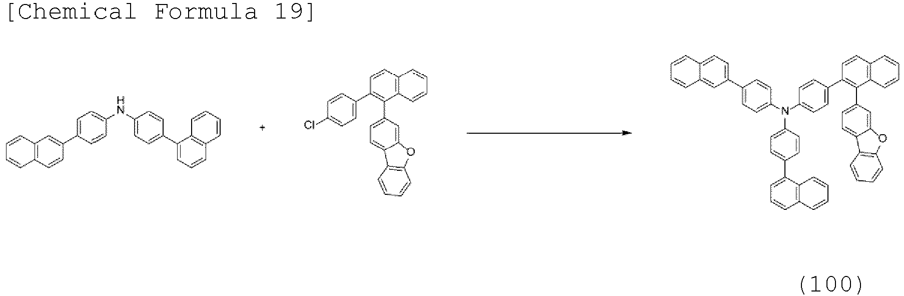

The structure of the obtained white powder was identified by NMR.

The ¹H-NMR (CDCl₃) detected 39 hydrogen signals, as follows.

δ (ppm) = 8.00-7.87 (12H), 7.70 (1H), 7.63 (1H), 7.58 (1H), 7.53-7.40 (12H), 7.38-7.25 (5H), 7.14-7.09 (6H), 7.00 (1H).

### Example 13

### <Synthesis of Compound (150)>

(biphenyl-4-yl)-[4-{1-(dibenzofuran-4-yl)naphthalene-2-yl}phenyl]amine: 15.0g, 9-(4-bromophenyl)phenanthrene: 10.2g, tris(dibenzylideneacetone)palladium(0): 0.5g, tri-tert-butylphosphine: 0.2g, and tert-butoxy sodium: 4.0g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a monochlorobenzene/acetone mixed solvent, whereby a white powder of Compound (150): 20.0g (yield: 90.7%) was obtained.

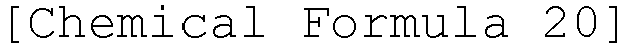

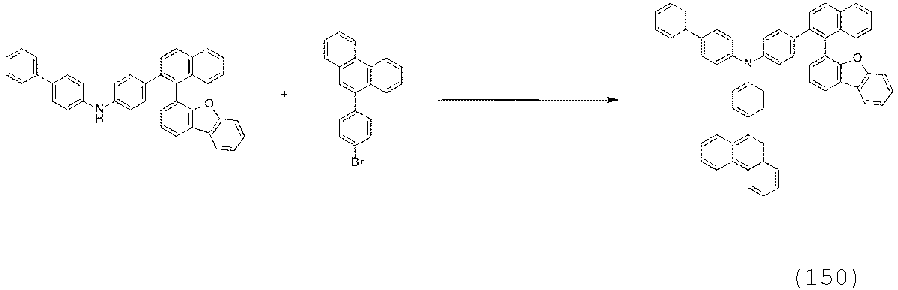

The structure of the obtained white powder was identified by NMR.

The ¹H-NMR (CDCl₃) detected 39 hydrogen signals, as follows.

δ (ppm) = 8.78 (1H), 8.76 (1H), 8.00-7.92 (5H), 7.87 (1H), 7.78 (1H), 7.70-7.24 (22H), 7.13-7.08 (6H), 6.99 (2H).

### Example 14

### <Synthesis of Compound (151)>

[4-{1-(dibenzofuran-4-yl)naphthalene-2-yl}phenyl]-phenylamine: 14.0g, 4-bromobiphenyl: 7.4g, tris(dibenzylideneacetone)palladium(0): 0.6g, tri-tert-butylphosphine: 0.3g, and tert-butoxy sodium: 4.4g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (151): 14.6g (yield: 78.4%) was obtained.

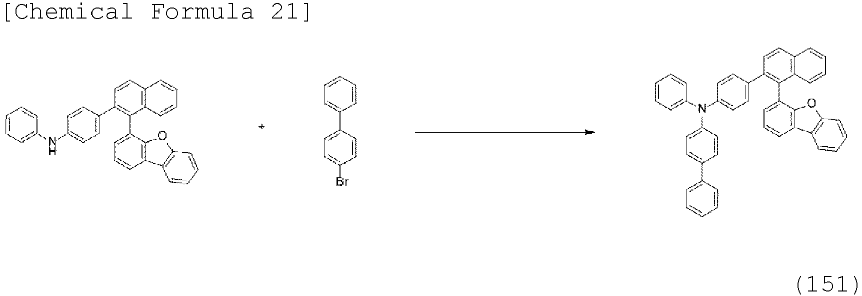

The structure of the obtained white powder was identified by NMR.

The ¹H-NMR (CDCl₃) detected 31 hydrogen signals, as follows.

δ (ppm) = 8.04 (1H), 8.00-7.93 (3H), 7.73-7.68 (2H), 7.53-7.50 (3H), 7.41-7.25 (11H), 7.12 (2H), 7.03 (2H), 6.94 (1H), 6.81-6.74 (6H).

### Example 15

### <Synthesis of Compound (102)>

[4-{1-(dibenzofuran-4-yl)naphthalene-2-yl}phenyl]-phenylamine: 13.0g, 1-(4-bromophenyl)naphthalene: 8.8g, tris(dibenzylideneacetone)palladium(0): 0.5g, tri-tert-butylphosphine: 0.2g, and tert-butoxy sodium: 4.1g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (102): 16.6g (yield: 88.8%) was obtained.

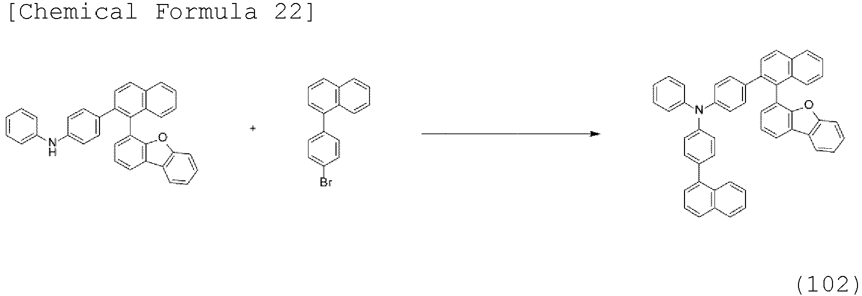

The structure of the obtained white powder was identified by NMR.

The ¹H-NMR (CDCl₃) detected 33 hydrogen signals, as follows.

δ (ppm) = 8.04 (1H), 7.98-7.89 (5H), 7.83 (1H), 7.75-7.69 (2H), 7.53-7.43 (4H), 7.40-7.29 (7H), 7.22 (2H), 7.15 (2H), 7.06 (2H), 7.95 (1H), 6.87-6.80 (6H).

### Example 16

### <Synthesis of Compound (104)>

[4-{1-(dibenzofuran-4-yl)naphthalene-2-yl}phenyl]-phenylamine: 13.0g, 9-(4-bromophenyl)phenanthrene: 10.3g, tris(dibenzylideneacetone)palladium(0): 0.5g, tri-tert-butylphosphine: 0.2g, and tert-butoxy sodium: 4.1g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (104): 16.1g (yield: 80.1%) was obtained.

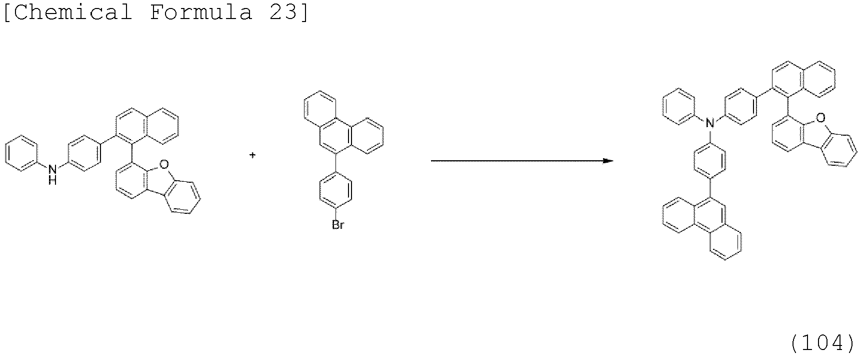

The structure of the obtained white powder was identified by NMR.

The ¹H-NMR (CDCl₃) detected 35 hydrogen signals, as follows.

δ (ppm) = 8.78 (1H), 8.72 (1H), 8.05 (1H), 8.00-7.94 (4H), 7.87 (1H), 7.74 (1H), 7.70-7.57 (6H), 7.50 (1H), 7.41-7.25 (8H), 7.16 (2H), 7.07 (2H), 6.96 (1H), 6.89-6.82 (6H).

### Example 17

### <Synthesis of Compound (106)>

Bis(biphenyl-4-yl)-{4-(4,4,5,5-tetramethyl-1,3,2-dioxaborolan-2-yl)phenyl]amine: 11.0g, 1-(dibenzofuran-4-yl)naphthalene-2-yl-trifluoromethanesulfonate: 13.0g, potassium carbonate: 6.9g, and tetrakistriphenylphosphine palladium(0): 0.6g were added into a reaction vessel, the mixture was refluxed and stirred in a toluene/ethanol/water mixed solvent overnight. After the reaction was completed, ethyl acetate was added thereto, an organic layer was collected by extraction and liquid separation, and then concentrated to obtain a crude product. The obtained crude product was purified by crystallization with a toluene/acetone mixed solvent, whereby a white powder of Compound (106): 11.5g (yield: 67.0%) was obtained.

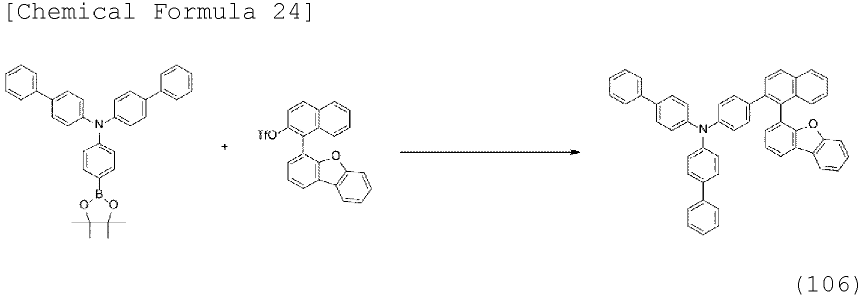

The structure of the obtained white powder was identified by NMR.

The ¹H-NMR (CDCl₃) detected 35 hydrogen signals, as follows.

δ (ppm) = 8.06 (1H), 8.01-7.94 (3H), 7.74-7.69 (2H), 7.55-7.48 (5H), 7.44-7.29 (16H), 7.06 (2H), 6.85-6.79 (6H).

### Example 18

### <Synthesis of Compound (152)>

[4-{1-(dibenzofuran-4-yl)naphthalene-2-yl}phenyl]-phenylamine: 7.0g, 2-bromo-9,9-diphenylfluorene: 6.6g, tert-butoxy sodium: 2.2g, tris(dibenzylideneacetone)palladium(0): 0.1g, and tri-tert-butylphosphine (50% toluene solution): 0.1g were added into a reaction vessel, the mixture was refluxed and stirred in a toluene solvent for three hours. After the reaction was completed, the insoluble matter was removed by filtration, then the resulting filtrate was concentrated to obtain a crude product. The obtained crude product was purified using column chromatography (support: silica gel, eluent: a dichloromethane/heptane mixed solvent), whereby a white powder of Compound(152): 7.4g (yield: 63.0%) was obtained.

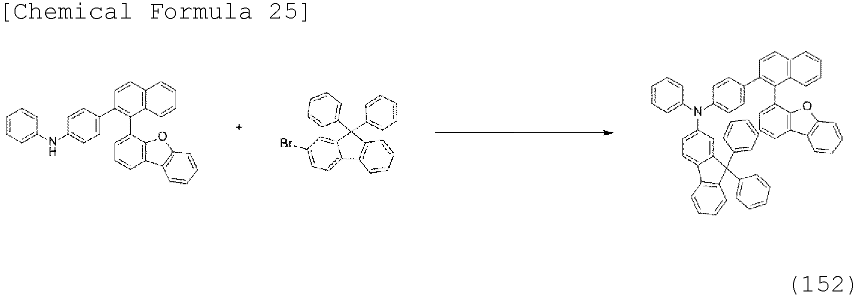

The structure of the obtained white powder was identified by NMR.

The ¹H-NMR (CDCl₃) detected 39 hydrogen signals, as follows.

δ (ppm) = 8.03 (1H), 7.95 (2H), 7.90 (1H), 7.70 (1H), 7.61 (2H), 7.49 (1H), 7.42-7.25 (9H), 7.19-7.01 (14H), 6.97 (2H), 6.88 (1H), 6.71 (4H), 6.89 (1H).

### Example 19

### <Synthesis of Compound (153)>

[4'-{(1-dibenzofuran-4-yl)naphthalene-2-yl}biphenyl-4-yl]-phenylamine: 10.0g, 1-(4-bromophenyl)naphthalene: 5.8g, tert-butoxy sodium: 2.7g, tris(dibenzylideneacetone)palladium(0): 0.2g, and tri-tert-butylphosphine: 0.2g were added into a reaction vessel, the mixture was refluxed and stirred in a toluene solvent for three hours. After the reaction was completed, the insoluble matter was removed by filtration, then the resulting filtrate was concentrated to obtain a crude product. The obtained crude product was purified using column chromatography (support: silica gel, eluent: a dichloromethane/heptane mixed solvent), whereby a white powder of Compound (153): 7.1g (yield: 51.5%) was obtained.

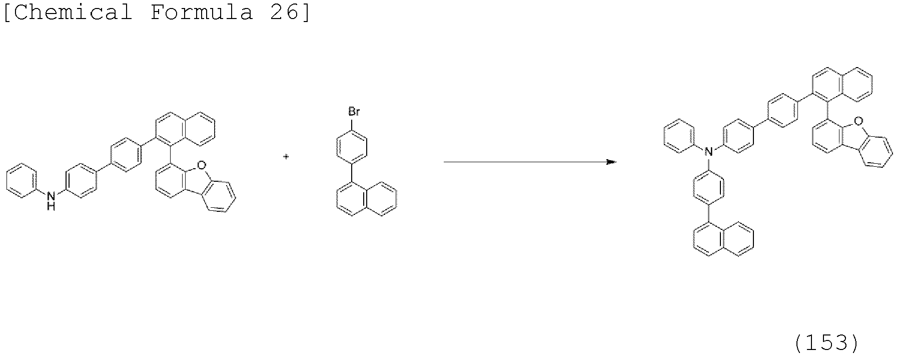

The structure of the obtained white powder was identified by NMR.

The ¹H-NMR (CDCl₃) detected 37 hydrogen signals, as follows.

δ (ppm) = 8.06-7.96 (4H), 7.90 (2H), 7.83 (1H), 7.72 (1H), 7.53-7.14 (28H), 7.05 (1H).

### Example 20

### <Synthesis of Compound (154)>

(biphenyl-4-yl)-[4-{1-(dibenzofuran-4-yl)naphthalene-2-yl}phenyl]amine: 13.0g, 2-(4-bromophenyl)naphthalene: 7.5g, tert-butoxy sodium: 3.5g, tris(dibenzylideneacetone)palladium(0): 0.4g, and tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (154): 15.1g (yield: 84.4%) was obtained.

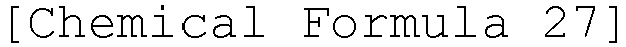

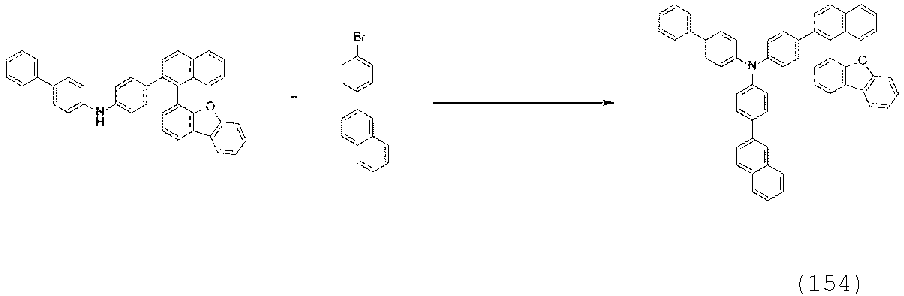

The structure of the obtained white powder was identified by NMR.

The ¹H-NMR (CDCl₃) detected 37 hydrogen signals, as follows.

δ (ppm) = 8.05 (1H), 8.01-7.95 (4H), 7.90-7.84 (3H), 7.74 (1H), 7.70 (2H), 7.55-7.31 (18H), 7.07 (2H), 6.90-6.81 (6H).

### Example 21

### <Synthesis of Compound (155)>

(biphenyl-4-yl)-[4-{1-(dibenzofuran-4-yl)naphthalene-2-yl}phenyl]amine: 13.0g, 1-(4-bromophenyl)naphthalene: 7.5g, tert-butoxy sodium: 3.5g, tris(dibenzylideneacetone)palladium(0): 0.4g, and tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (155): 17.6g (yield: 98.4%) was obtained.

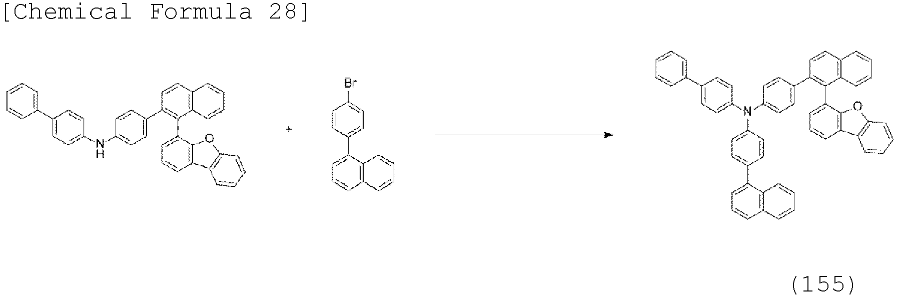

The structure of the obtained white powder was identified by NMR.

The ¹H-NMR (CDCl₃) detected 37 hydrogen signals, as follows.

δ (ppm) = 8.06 (1H), 7.98-7.89 (5H), 7.84 (1H), 7.76-7.70 (2H), 7.56-7.24 (20H), 7.09 (2H), 6.91-6.85 (6H).

### Example 22

### <Synthesis of Compound (156)>

(biphenyl-4-yl)-[4-{1-(dibenzofuran-4-yl)naphthalene-2-yl}phenyl]amine: 12.6g, 9-(4-bromophenyl)phenanthrene: 8.6g, tert-butoxy sodium: 3.4g, tris(dibenzylideneacetone)palladium(0): 0.4g, and tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (156): 6.8g (yield: 36.7%) was obtained.

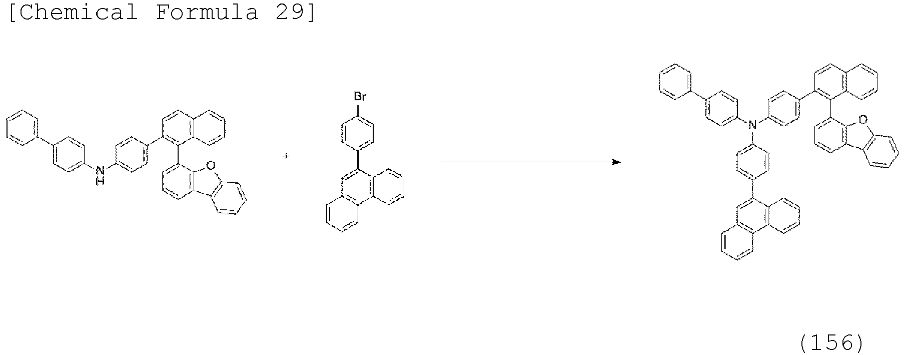

The structure of the obtained white powder was identified by NMR.

The ¹H-NMR (CDCl₃) detected 39 hydrogen signals, as follows.

δ (ppm) = 8.79 (1H), 8.72 (1H), 8.00 (1H), 8.02-7.94 (4H), 7.89 (1H), 7.76 (1H), 7.72-7.29 (22H), 7.10 (2H), 6.93-6.87 (6H).

### Example 23

### <Synthesis of Compound (157)>

{4-(naphthalene-1-yl)phenyl}-{4-(naphthalene-2-yl)phenyl}amine: 10.0g, 1-chloro-4-{1-(dibenzofuran-4-yl)naphthalene-2-yl}benzene: 10.3g, tert-butoxy sodium: 3.4g, tris(dibenzylideneacetone)palladium(0): 0.4g, and tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (157): 18.6g (yield: 99.3%) was obtained.

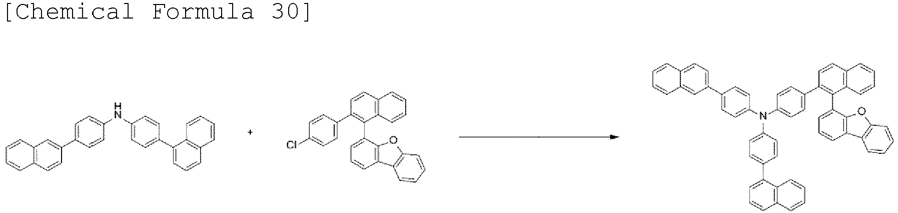

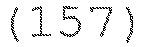

The structure of the obtained white powder was identified by NMR.

The ¹H-NMR (CDCl₃) detected 39 hydrogen signals, as follows.

δ (ppm) = 8.06 (1H), 7.99-7.95 (5H), 7.92-7.84 (5H), 7.76 (1H), 7.71 (2H), 7.52-7.47 (8H), 7.43-7.33 (7H), 7.27 (2H), 7.10 (2H), 6.96-6.88 (6H).

### Example 24

### <Synthesis of Compound (158)>

[4-{(1-dibenzofuran-4-yl)naphthalene-2-yl}phenyl]-phenylamine: 8.1g, 9-(4'-chlorobiphenyl-3-yl)-9H-carbazole: 6.8g, tert-butoxy sodium: 2.5g, tris(dibenzylideneacetone)palladium(0): 0.3g, and tri-tert-butylphosphine: 0.1g were added into a reaction vessel, the mixture was refluxed and stirred In a toluene solvent overnight. After the reaction was completed, the insoluble matter was removed by filtration, then the resulting filtrate was concentrated to obtain a crude product. The obtained crude product was purified using column chromatography (support: silica gel, eluent: a dichloromethane/heptane mixed solvent), whereby a white powder of Compound (158): 9.8g (yield: 71.7%) was obtained.

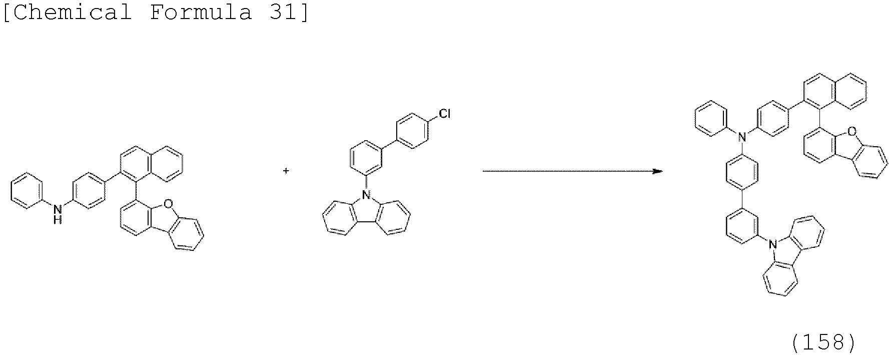

The structure of the obtained white powder was identified by NMR.

The ¹H-NMR (CDCl₃) detected 38 hydrogen signals, as follows.

δ (ppm) = 8.17 (2H), 8.04 (1H), 7.94-7.92 (3H), 7.73-7.63 (5H), 7.49-7.29 (16H), 7.13 (2H), 7.04 (2H), 6.95 (1H), 6.82-6.75 (6H).

### Example 25

### <Synthesis of Compound (159)>

(biphenyl-4-yl)-[4-{1-(dibenzofuran-4-yl)naphthalene-2-yl}phenyl]amine: 9.0g, 2-(4-chlorophenyl)dibenzofuran: 5.1g, tert-butoxy sodium: 2.4g, tris(dibenzylideneacetone)palladium(0): 0.2g, and tri-tert-butylphosphine: 0.1g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (159): 11.2g (yield: 85.5%) was obtained.

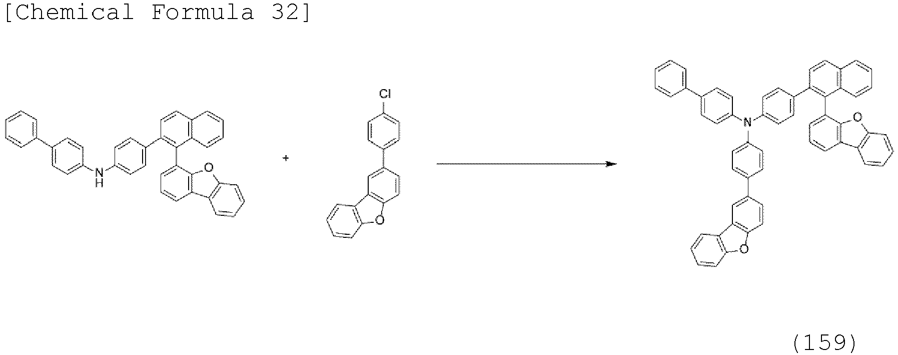

The structure of the obtained white powder was identified by NMR.

The ¹H-NMR (CDCl₃) detected 37 hydrogen signals, as follows.

δ (ppm) = 8.09-7.96 (6H), 7.75 (1H), 7.70 (1H), 7.65-7.30 (21H), 7.08 (2H), 6.92-6.82 (6H).

### Example 26

### <Synthesis of Compound (160)>

(biphenyl-4-yl)-[4-{1-(dibenzofuran-4-yl)naphthalene-2-yl}phenyl]amine: 9.0g, 3-(4-bromophenyl)dibenzofuran: 6.0g, tert-butoxy sodium: 2.4g, tris(dibenzylideneacetone)palladium (0): 0.2g, and tri-tert-butylphosphine: 0.1g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/acetone mixed solvent, whereby a white powder of Compound (160): 11.1g (yield: 85.5%) was obtained.

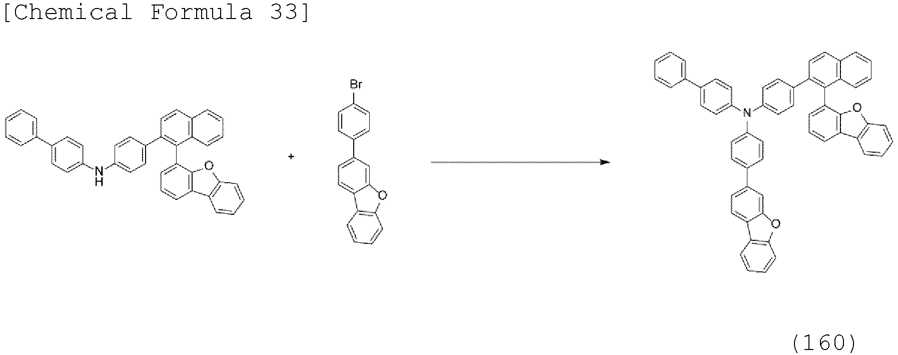

The structure of the obtained white powder was identified by NMR.

The ¹H-NMR (CDCl₃) detected 37 hydrogen signals, as follows.

δ (ppm) = 8.05 (1H), 8.01 (1H), 7.98-7.95 (4H), 7.75-7.69 (3H), 7.59-7.29 (21H), 7.08 (2H), 6.87-6.83 (5H).

### Example 27

### <Synthesis of Compound (161)>

(biphenyl-4-yl)-[4-{1-(dibenzofuran-4-yl)naphthalene-2-yl}phenyl]amine: 9.0g, 4-bromo-[1,1':4',1"]terphenyl: 5.7g, tert-butoxy sodium: 2.4g, tris(dibenzylideneacetone)palladium(0): 0.2g, and tri-tert-butylphosphine: 0.1g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified using column chromatography (support: silica gel, eluent: a dichloromethane/heptane mixed solvent), whereby a white powder of Compound (161): 8.0g (yield: 62.5%) was obtained.

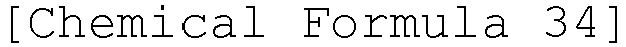

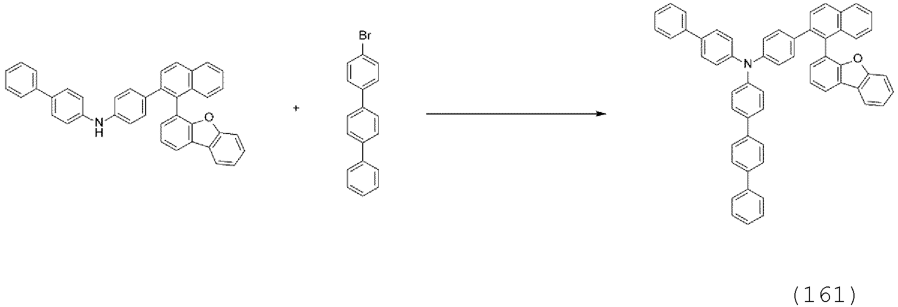

The structure of the obtained white powder was identified by NMR.

The ¹H-NMR (CDCl₃) detected 39 hydrogen signals, as follows.

δ (ppm) = 8.05 (1H), 8.02-7.93 (3H), 7.75-7.61 (8H), 7.56-7.29 (19H), 7.07 (2H), 6.86-6.80 (6H).

### Example 28

### <Synthesis of Compound (162)>

[4-{1-(dibenzofuran-3-yl)naphthalene-2-yl}phenyl]-phenylamine: 13.3g, 9-(4'-chlorobiphenyl-3-yl)-9H-carbazole: 9.6g, tert-butoxy sodium: 3.6g, tris(dibenzylideneacetone)palladium(0): 0.5g, and tri-tert-butylphosphine: 0.2g were added into a reaction vessel, the mixture was refluxed and stirred in a toluene solvent overnight. After the reaction was completed, the insoluble matter was removed by filtration, then the resulting filtrate was concentrated to obtain a crude product. The obtained crude product was purified by crystallization with a toluene/heptane mixed solvent, whereby a white powder of Compound (162): 18.8g (yield: 97.6%) was obtained.

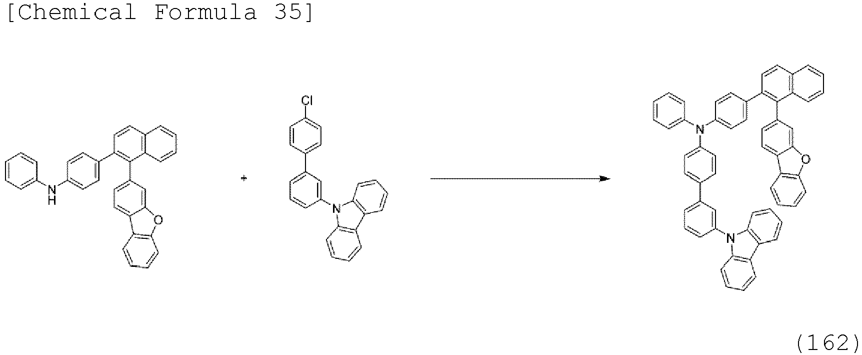

The structure of the obtained white powder was identified by NMR.

The ¹H-NMR (CDCl₃) detected 38 hydrogen signals, as follows.

δ (ppm) = 8.17 (2H), 7.97-7.87 (4H), 7.76 (1H), 7.67-7.60 (3H), 7.55-7.39 (10H), 7.34-7.13 (8H), 7.08-7.01 (5H), 6.99-6.91 (3H), 6.87 (2H).

### Example 29

The melting points and glass transition points of the arylamine compounds of the general formula (1) or (2) were determined using a high-sensitive differential scanning calorimeter (DSC3100SA produced by Bruker AXS). The measurement results are summarized in Table 1.

**[Table 1]**

| | Melting point | Glass transition point |
|---|---|---|
| Compound 91 | 215°C | 111°C |
| Compound 93 | - | 118°C |
| Compound 97 | - | 125°C |
| Compound 132 | 263°C | 131°C |
| Compound 146 | - | 143°C |
| Compound 103 | 225°C | 114°C |
| Compound 85 | - | 133°C |
| Compound 147 | - | 142°C |
| Compound 148 | - | 144°C |
| Compound 95 | - | 120°C |
| Compound 149 | - | 130°C |
| Compound 100 | - | 130°C |
| Compound 150 | - | 141°C |
| Compound 151 | - | 105°C |
| Compound 102 | 234°C | 116°C |
| Compound 104 | - | 135°C |
| Compound 106 | - | 123°C |
| Compound 152 | - | 143°C |
| Compound 153 | - | 133°C |
| Compound 154 | - | 127°C |
| Compound 155 | - | 130°C |
| Compound 156 | - | 147°C |
| Compound 157 | - | 135°C |
| Compound 158 | - | 138°C |
| Compound 159 | - | 133°C |
| Compound 160 | - | 134°C |
| Compound 161 | - | 134°C |
| Compound 162 | - | 136°C |

The arylamine compounds of the general formula (1) or (2) have glass transition points of 100 °C or higher, demonstrating that the compounds have a stable thin-film state.

### Example 30

A 100 nm-thick vapor-deposited film was fabricated on an ITO substrate using the arylamine compounds of the general formula (1) or (2), and a work function (eV) was measured using an ionization potential measuring device (PYS-202 produced by Sumitomo Heavy Industries, Ltd.). The measurement results are summarized in Table 2.

**[Table 2]**

| | Work function |
|---|---|
| Compound 91 | -5.63 |
| Compound 93 | -5.66 |
| Compound 97 | -5.68 |
| Compound 132 | -5.62 |
| Compound 146 | -5.68 |
| Compound 103 | -5.64 |
| Compound 85 | -5.61 |
| Compound 147 | -5.60 |
| Compound 148 | -5.61 |
| Compound 95 | -5.61 |
| Compound 149 | -5.70 |
| Compound 100 | -5.61 |
| Compound 150 | -5.62 |
| Compound 151 | -5.64 |
| Compound 102 | -5.66 |
| Compound 104 | -5.66 |
| Compound 106 | -5.61 |
| Compound 152 | -5.63 |
| Compound 153 | -5.66 |
| Compound 154 | -5.62 |
| Compound 155 | -5.62 |
| Compound 156 | -5.62 |
| Compound 157 | -5.62 |
| Compound 158 | -5.67 |
| Compound 159 | -5.60 |
| Compound 160 | -5.62 |
| Compound 161 | -5.63 |
| Compound 162 | -5.67 |

As the results show, the arylamine compounds of the general formula (1) or (2) have desirable energy levels compared to the work function 5.4 eV of common hole transport materials such as NPD and TPD, and thus possess desirable hole transportability and an excellent electron blocking ability.

### Example 31

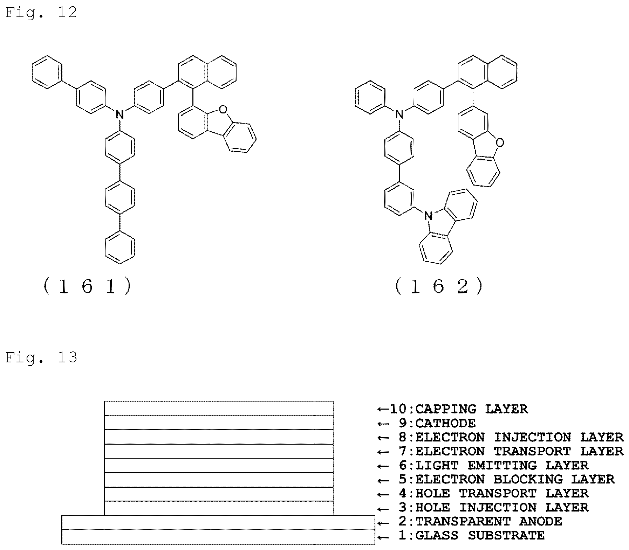

The organic EL device, as shown in FIG. 13, was fabricated by vapor-depositing a hole injection layer 3, a hole transport layer 4, an electron blocking layer 5, a light emitting layer 6, an electron transport layer 7, an electron injection layer 8, and a cathode 9, a capping layer 10 in this order on a glass substrate 1 on which a reflective ITO electrode was formed as a transparent anode 2 beforehand.

Specifically, as the transparent anode 2, an ITO film with a thickness of 50 nm, a silver alloy reflective film with a thickness of 100 nm, and an ITO film with a thickness of 5 nm were deposited on the glass substrate 1 in this order. The glass substrate 1 with ITO was subjected to ultrasonic washing in isopropyl alcohol for 20 minutes and then dried for 10 minutes on a hot plate heated to 250°C. After UV ozone treatment for 2 minutes, the glass substrate with ITO was installed in a vacuum vapor deposition apparatus, and the pressure was reduced to 0.001 Pa or lower.

Subsequently, as the hole injection layer 3 covering the transparent anode 2, an electron acceptor (Acceptor-1) of the structural formula below and Compound (HTM-1) of the structural formula below were formed in a film thickness of 10 nm by dual vapor deposition at a vapor deposition rate ratio of Acceptor-1:Compound (HTM-1) = 3:97.

The hole transport layer 4 was formed on the hole injection layer 3 by forming Compound (HTM-1) of the structural formula below in a film thickness of 140 nm.

The electron blocking layer 5 was formed on the hole transport layer 4 by forming Compound (91) of Example 1 in a film thickness of 5 nm.

Then, the light emitting layer 6 was formed on the electron blocking layer 5 in a film thickness of 20 nm by dual vapor deposition of Compound (EMD-1) of the structural formula below and Compound (EMH-1) of the structural formula below at a vapor deposition rate ratio of Compound (EMD-1) : Compound (EMH-1) = 5:95.

The electron transport layer 7 was formed on the light emitting layer 6 in a film thickness of 30 nm by dual vapor deposition of Compound (ETM-1) of the structural formula below and Compound (ETM-2) of the structural formula below at a vapor deposition rate ratio of Compound (ETM-1) : Compound (ETM-2) = 50:50.

The electron injection layer 8 was formed on the electron transport layer 7 by forming lithium fluoride in a film thickness of 1 nm.

The cathode 9 was formed on the electron injection layer 8 by forming a magnesium silver alloy in a film thickness of 12 nm.

Finally, the capping layer 10 was formed by forming Compound (CPL-1) of the structural formula below in a film thickness of 60 nm.

The characteristics of the thus fabricated organic EL device were measured in the atmosphere at an ordinary temperature.

Table 3 summarizes the results of the emission characteristics measurements performed by applying a DC voltage to the fabricated organic EL device.

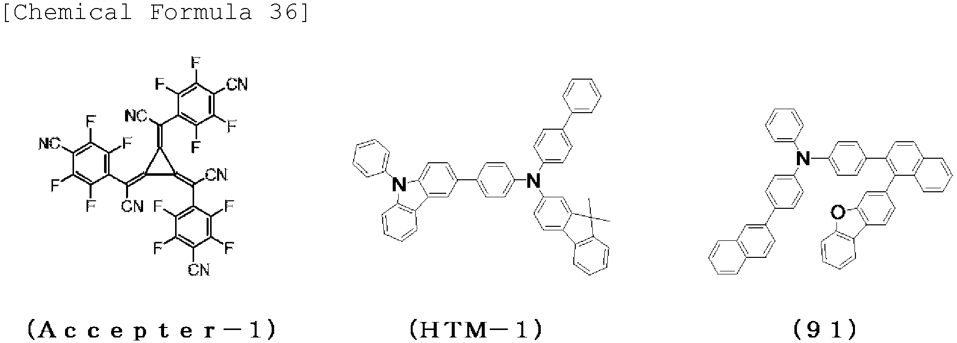

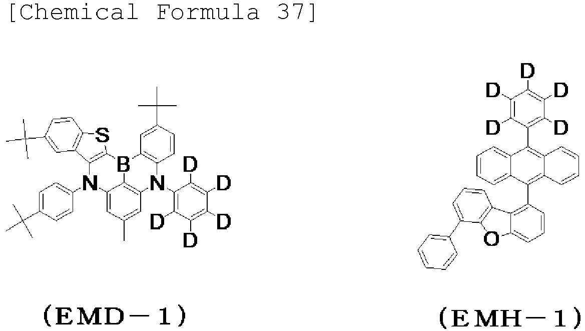

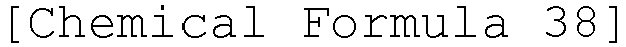

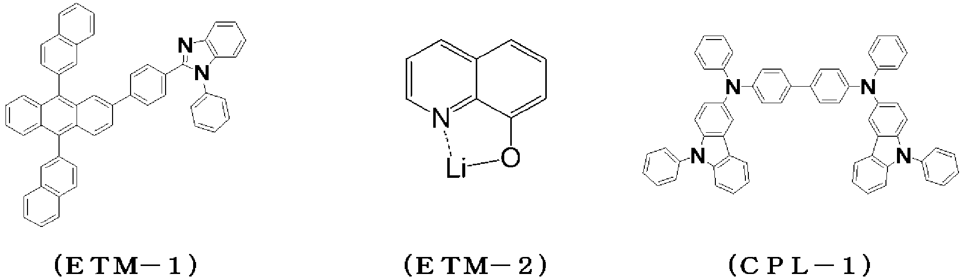

### Example 32

### Examples 32 to 58

An organic EL devices were fabricated under the same conditions as in Example 31 except that the electron blocking layers 5 were formed by using the compounds obtained in Examples 2 to 28 instead of Compound (91) of Example 1. The characteristics of the thus fabricated organic EL device were measured in the atmosphere at an ordinary temperature. Table 3 summarizes the results of emission characteristics measurements performed by applying a DC voltage to the fabricated organic EL device.

### Comparative Example 1

For comparison, an organic EL device was fabricated under the same conditions as in Example 31 except that the electron blocking layer 5 was formed by using Compound (HTM-2) (refer to Patent Document 7, for example) of the structural formula below instead of Compound (91) of Example 1. The characteristics of the thus fabricated organic EL device were measured in the atmosphere at an ordinary temperature. Table 3 summarizes the results of emission characteristics measurements performed by applying a DC voltage to the fabricated organic EL device.

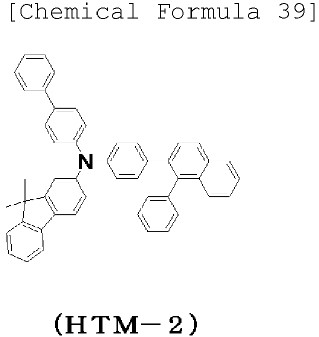

### Comparative Example 2

For comparison, an organic EL device was fabricated under the same conditions as in Example 31 except that the electron blocking layer 5 was formed by using Compound (HTM-3) (refer to Patent Document 8, for example) of the structural formula below instead of Compound (91) of Example 1.

The characteristics of the thus fabricated organic EL device were measured in the atmosphere at an ordinary temperature. Table 3 summarizes the results of emission characteristics measurements performed by applying a DC voltage to the fabricated organic EL device.

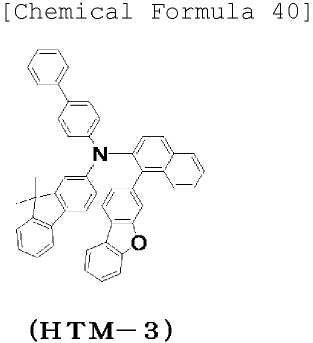

The device lifetime of the fabricated organic EL devices in Examples 31 to 58 and Comparative Examples 1 to 2 were measured. Table 3 summarizes the results of the device lifetime measurements. The device lifetime was measured as the time elapsed until the emission luminance of 1,000 cd/m² (initial luminance) at the start of emission was attenuated to 950 cd/m² (corresponding to 95% when taking the initial luminance as 100%, attenuation to 95%) when carrying out constant current driving.

**[Table 3]**

| | Electron blocking layer | Voltage [V] | Luminance [ cd/m² ] | Luminous efficiency [cd/A] | Power efficiency [lm/W] | Device lifetime |
|---|---|---|---|---|---|---|
| | | (@10mA/cm²) | | | | Attenuation to 95% |
| Ex.31 | Cpd. 91 | 3.28 | 1007 | 10.07 | 9.85 | 435hr. |
| Ex.32 | Cpd. 93 | 3.32 | 1004 | 10.05 | 9.53 | 359hr. |
| Ex.33 | Cpd. 97 | 3.26 | 985 | 9.85 | 9.50 | 540hr. |
| Ex.34 | Cpd. 132 | 3.29 | 975 | 9.77 | 9.40 | 416hr. |
| Ex.35 | Cpd. 146 | 3.28 | 1022 | 10.24 | 9.81 | 335hr. |
| Ex.36 | Cpd. 103 | 3.23 | 983 | 9.84 | 9.57 | 467hr. |
| Ex.37 | Cpd. 85 | 3.27 | 971 | 9.74 | 9.37 | 339hr. |
| Ex.38 | Cpd. 147 | 3.31 | 1013 | 10.13 | 9.61 | 335hr. |
| Ex.39 | Cpd. 148 | 3.28 | 940 | 9.41 | 9.02 | 341hr. |
| Ex.40 | Cpd. 95 | 3.26 | 979 | 9.82 | 9.48 | 426hr. |
| Ex.41 | Cpd. 149 | 3.26 | 1008 | 10.11 | 9.72 | 386hr. |
| Ex.42 | Cpd. 100 | 3.27 | 993 | 9.95 | 9.57 | 396hr. |
| Ex.43 | Cpd. 150 | 3.29 | 991 | 9.93 | 9.50 | 354hr. |
| Ex.44 | Cpd. 151 | 3.22 | 880 | 8.81 | 8.59 | 361hr. |
| Ex.45 | Cpd. 102 | 3.23 | 895 | 8.96 | 8.91 | 349hr. |
| Ex.46 | Cpd. 104 | 3.23 | 876 | 8.77 | 8.55 | 347hr. |
| Ex.47 | Cpd. 106 | 3.24 | 1002 | 10.05 | 9.70 | 348hr. |
| Ex.48 | Cpd. 152 | 3.24 | 946 | 9.48 | 9.21 | 414hr. |
| Ex.49 | Cpd. 153 | 3.23 | 900 | 9.01 | 8.95 | 392hr. |
| Ex.50 | Cpd. 154 | 3.24 | 934 | 9.34 | 9.07 | 482hr. |
| Ex.51 | Cpd. 155 | 3.24 | 888 | 8.89 | 8.63 | 442hr. |
| Ex.52 | Cpd. 156 | 3.25 | 886 | 8.87 | 8.57 | 432hr. |
| Ex.53 | Cpd. 157 | 3.22 | 877 | 8.78 | 8.56 | 382hr. |
| Ex.54 | Cpd. 158 | 3.32 | 917 | 9.20 | 8.72 | 412hr. |
| Ex.55 | Cpd. 159 | 3.22 | 893 | 8.93 | 8.66 | 391hr. |
| Ex.56 | Cpd. 160 | 3.23 | 919 | 9.20 | 8.94 | 455hr. |
| Ex.57 | Cpd. 161 | 3.24 | 877 | 8.79 | 8.55 | 363hr. |
| Ex.58 | Cpd. 162 | 3.34 | 982 | 9.86 | 9.29 | 365hr. |
| Com. Ex.1 | HTM-2 | 3.44 | 915 | 9.15 | 8.88 | 253hr. |
| Com. Ex.2 | HTM-3 | 3.36 | 875 | 8.76 | 8.54 | 334hr. |

As shown in Table 3, the luminous efficiency upon passing a current with a current density of 10 mA/cm² was 8.77 to 10.24 cd/A for the organic EL devices in Examples 31 to 58, which was higher than 8.76 to 9.15 cd/A for the organic EL devices in Comparative Examples 1 to 2. Further, the power efficiency was 8.55 to 9.85 lm/W for the organic EL devices in Examples 31 to 58, which was higher than 8.54 to 8.88 lm/W for the organic EL devices in Comparative Examples 1 to 2. Table 3 also shows that the device lifetime (attenuation to 95%) was 335 to 540 hours for the organic EL devices in Examples 31 to 58, showing achievement of a far longer lifetime than 253 to 334 hours for the organic EL devices in Comparative Examples 1 to 2.

As is clear from the above results, it was found that the organic EL device of the present invention can achieve an organic EL device having high luminous efficiency and a long lifetime by using the arylamine compound having greater hole mobility, and superior electron blocking ability compared to the conventional organic EL devices.

### Industrial Applicability

In the organic EL device of the present invention in which an arylamine compound having a specific structure is using, luminous efficiency can be improved, and also durability of the organic EL device can be improved to attain potential applications for, for example, home electric appliances and illuminations.

### Reference Signs List

- 1: Glass substrate
- 2: Transparent anode
- 3: Hole injection layer
- 4: Hole transport layer
- 5: Electron blocking layer
- 6: Light emitting layer
- 7: Electron transport layer
- 8: Electron injection layer
- 9: Cathode
- 10: Capping layer

## Claims

1. An arylamine compound of the following general formula (1) or (2): 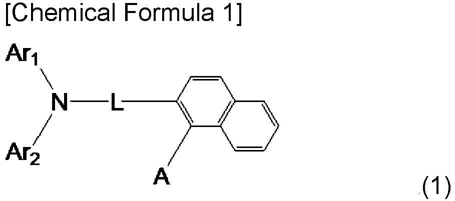
wherein Ar₁ and Ar₂ may be the same or different, and represent a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted indenyl group, a substituted or unsubstituted pyrenyl group, a substituted or unsubstituted perylenyl group, a substituted or unsubstituted fluoranthenyl group, a substituted or unsubstituted triphenylenyl group, a substituted or unsubstituted pyridyl group, a substituted or unsubstituted pyrimidinyl group, a substituted or unsubstituted triazinyl group, a substituted or unsubstituted furyl group, a substituted or unsubstituted pyrrolyl group, a substituted or unsubstituted thienyl group, a substituted or unsubstituted quinolyl group, a substituted or unsubstituted isoquinolyl group, a substituted or unsubstituted benzofuranyl group, a substituted or unsubstituted benzothienyl group, a substituted or unsubstituted indolyl group, a substituted or unsubstituted carbazolyl group, a substituted or unsubstituted benzoxazolyl group, a substituted or unsubstituted benzothiazolyl group, a substituted or unsubstituted azafluorenyl group, a substituted or unsubstituted diazafluorenyl group, a substituted or unsubstituted azaspirobifluorenyl group, a substituted or unsubstituted diazaspirobifluorenyl group, a substituted or unsubstituted quinoxalinyl group, a substituted or unsubstituted benzoimidazolyl group, a substituted or unsubstituted pyrazolyl group, a substituted or unsubstituted dibenzofuranyl group, a substituted or unsubstituted dibenzothienyl group, a substituted or unsubstituted naphthyridinyl group, a substituted or unsubstituted phenanthrolinyl group, a substituted or unsubstituted acridinyl group, or a substituted or unsubstituted carbolinyl group, when Ar₁ and Ar₂ have substituents, the substituents are a deuterium atom, a cyano group, a nitro group, a halogen atom, a silyl group, a linear or branched alkyl group of 1 to 6 carbon atoms, a linear or branched alkyloxy group of 1 to 6 carbon atoms, an alkenyl group, an aryloxy group, an arylalkyloxy group, a phenyl group, a biphenyl group, a terphenyl group, a naphthyl group, an anthracenyl group, a phenanthrenyl group, an indenyl group, a pyrenyl group, a perylenyl group, a fluoranthenyl group, a triphenylenyl group, or an aromatic heterocyclic group;
L represents a substituted or unsubstituted divalent group of an aromatic hydrocarbon, a substituted or unsubstituted divalent group of an aromatic heterocyclic ring, or a substituted or unsubstituted divalent group of condensed polycyclic aromatics;
A represents a substituted or unsubstituted naphthyl group, a substituted or unsubstituted dibenzofuranyl group, a substituted or unsubstituted dibenzothienyl group, a substituted or unsubstituted triphenylsilyl group, or a substituted or unsubstituted phenanthrenyl group, 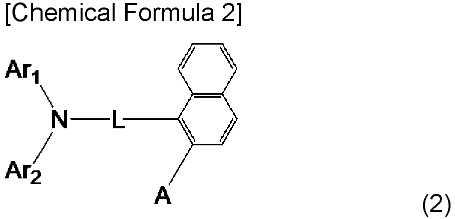
wherein, Ar₁, Ar₂, L, and A are as defined in the general formula (1).

2. The arylamine compound according to claim 1, wherein, in the general formula (1) or (2), Ar₁ and/or Ar₂ is a substituted or unsubstituted phenyl group, a substituted or unsubstituted biphenyl group, a substituted or unsubstituted naphthyl group, a substituted or unsubstituted dibenzofuranyl group, a substituted or unsubstituted dibenzothienyl group, or a substituted or unsubstituted phenanthrenyl group.

3. The arylamine compound according to claim 1 or 2, wherein, in the general formula (1) or (2), L is an unsubstituted phenylene group, an unsubstituted naphthylene group, or an unsubstituted biphenylene group.

4. The arylamine compound according to any one of claims 1 to 3, wherein, in the general formula (1) or (2), A is an unsubstituted naphthyl group, an unsubstituted dibenzofuranyl group, an unsubstituted dibenzothienyl group, an unsubstituted triphenyl silyl group, or an unsubstituted phenanthrenyl group.

5. The arylamine compound according to any one of claims 1 to 4, wherein, in the general formula (1) or (2), A is an unsubstituted 1-naphthyl group, an unsubstituted 2-a naphthyl group, an unsubstituted 1-dibenzofuranyl group, an unsubstituted 2-dibenzofuranyl group, an unsubstituted 3-dibenzofuranyl group, an unsubstituted 4-dibenzofuranyl group, an unsubstituted 3-dibenzothienyl group, an unsubstituted 4-dibenzothienyl group, or an unsubstituted 9-phenanthrenyl group.

6. An organic electroluminescent device including a pair of electrodes and at least one organic layer sandwiched therebetween, wherein the organic layer includes the arylamine compound of any one of claims 1 to 5.

7. The organic electroluminescent device according to claim 6, wherein the organic layer is a hole transport layer.

8. The organic electroluminescent device according to claim 6, wherein the organic layer is an electron blocking layer.

9. The organic electroluminescent device according to claim 6, wherein the organic layer is a hole injection layer.

10. The organic electroluminescent device according to claim 6, wherein the organic layer is a light emitting layer.

11. An electronic apparatus including a pair of electrodes and at least one organic layer sandwiched therebetween, wherein the organic layer includes the arylamine compound according to any one of claims 1 to 5.

## Patentansprüche

1. Arylaminverbindung der folgenden allgemeinen Formel (1) oder (2): 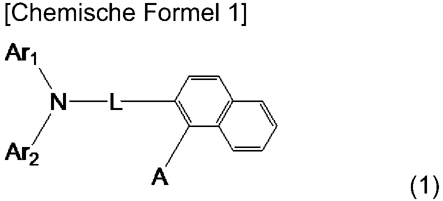
wobei Ar₁ und Ar₂ gleich oder verschieden sein können und eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Phenanthrenylgruppe, eine substituierte oder unsubstituierte Indenylgruppe, eine substituierte oder unsubstituierte Pyrenylgruppe, eine substituierte oder unsubstituierte Perylenylgruppe, eine substituierte oder unsubstituierte Fluoranthenylgruppe, eine substituierte oder unsubstituierte Triphenylenylgruppe, eine substituierte oder unsubstituierte Pyridylgruppe, eine substituierte oder unsubstituierte Pyrimidinylgruppe, eine substituierte oder unsubstituierte Triazinylgruppe, eine substituierte oder unsubstituierte Furylgruppe, eine substituierte oder unsubstituierte Pyrrolylgruppe, eine substituierte oder unsubstituierte Thienylgruppe, eine substituierte oder unsubstituierte Chinolylgruppe, eine substituierte oder unsubstituierte Isochinolylgruppe, eine substituierte oder unsubstituierte Benzofuranylgruppe, eine substituierte oder unsubstituierte Benzothienylgruppe, eine substituierte oder unsubstituierte Indolylgruppe, eine substituierte oder unsubstituierte Carbazolylgruppe, eine substituierte oder unsubstituierte Benzoxazolylgruppe, eine substituierte oder unsubstituierte Benzothiazolylgruppe, eine substituierte oder unsubstituierte Azafluorenylgruppe, eine substituierte oder unsubstituierte Diazafluorenylgruppe, eine substituierte oder unsubstituierte Azaspirobifluorenylgruppe, eine substituierte oder unsubstituierte Diazaspirobifluorenylgruppe, eine substituierte oder unsubstituierte Chinoxalinylgruppe, eine substituierte oder unsubstituierte Benzoimidazolylgruppe, eine substituierte oder unsubstituierte Pyrazolylgruppe, eine substituierte oder unsubstituierte Dibenzofuranylgruppe, eine substituierte oder unsubstituierte Dibenzothienylgruppe, eine substituierte oder unsubstituierte Naphthyridinylgruppe, eine substituierte oder unsubstituierte Phenanthrolinylgruppe, eine substituierte oder unsubstituierte Acridinylgruppe oder eine substituierte oder unsubstituierte Carbolinylgruppe darstellen, wobei, wenn Ar₁ und Ar₂ Substituenten aufweisen, die Substituenten ein Deuteriumatom, eine Cyanogruppe, eine Nitrogruppe, ein Halogenatom, eine Silylgruppe, eine lineare oder verzweigte Alkylgruppe mit 1 bis 6 Kohlenstoffatomen, eine lineare oder verzweigte Alkyloxygruppe mit 1 bis 6 Kohlenstoffatomen, eine Alkenylgruppe, eine Aryloxygruppe, eine Arylalkyloxygruppe, eine Phenylgruppe, eine Biphenylgruppe, eine Terphenylgruppe, eine Naphthylgruppe, eine Anthracenylgruppe, eine Phenanthrenylgruppe, eine Indenylgruppe, eine Pyrenylgruppe, eine Perylenylgruppe, eine Fluoranthenylgruppe, eine Triphenylenylgruppe oder eine aromatische heterocyclische Gruppe sind;
wobei L eine substituierte oder unsubstituierte zweiwertige Gruppe eines aromatischen Kohlenwasserstoffs, eine substituierte oder unsubstituierte zweiwertige Gruppe eines aromatischen heterocyclischen Rings oder eine substituierte oder unsubstituierte zweiwertige Gruppe von kondensierten polyzyklischen Aromaten darstellt;
wobei A eine substituierte oder unsubstituierte Naphthylgruppe, eine substituierte oder unsubstituierte Dibenzofuranylgruppe, eine substituierte oder unsubstituierte Dibenzothienylgruppe, eine substituierte oder unsubstituierte Triphenylsilylgruppe oder eine substituierte oder unsubstituierte Phenanthrenylgruppe darstellt, 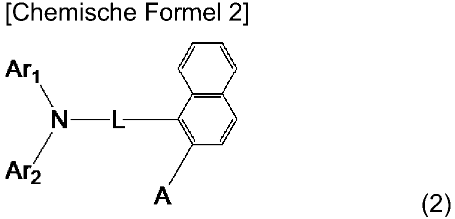
wobei Ar₁, Ar₂, L und A wie in der allgemeinen Formel (1) definiert sind.

2. Arylaminverbindung nach Anspruch 1, wobei in der allgemeinen Formel (1) oder (2) Ar₁ und/oder Ar₂ eine substituierte oder unsubstituierte Phenylgruppe, eine substituierte oder unsubstituierte Biphenylgruppe, eine substituierte oder unsubstituierte Naphthylgruppe, eine substituierte oder unsubstituierte Dibenzofuranylgruppe, eine substituierte oder unsubstituierte Dibenzothienylgruppe oder eine substituierte oder unsubstituierte Phenanthrenylgruppe ist.

3. Arylaminverbindung nach Anspruch 1 oder 2, wobei in der allgemeinen Formel (1) oder (2) L eine unsubstituierte Phenylengruppe, eine unsubstituierte Naphthylengruppe oder eine unsubstituierte Biphenylengruppe ist.

4. Arylaminverbindung nach einem der Ansprüche 1 bis 3, wobei in der allgemeinen Formel (1) oder (2) A eine unsubstituierte Naphthylgruppe, eine unsubstituierte Dibenzofuranylgruppe, eine unsubstituierte Dibenzothienylgruppe, eine unsubstituierte Triphenylsilylgruppe oder eine unsubstituierte Phenanthrenylgruppe ist.

5. Arylaminverbindung nach einem der Ansprüche 1 bis 4, wobei in der allgemeinen Formel (1) oder (2) A eine unsubstituierte 1-Naphthylgruppe, eine unsubstituierte 2-a-Naphthylgruppe, eine unsubstituierte 1-Dibenzofuranylgruppe, eine unsubstituierte 2-Dibenzofuranylgruppe, eine unsubstituierte 3-Dibenzofuranylgruppe, eine unsubstituierte 4-Dibenzofuranylgruppe, eine unsubstituierte 3-Dibenzothienylgruppe, eine unsubstituierte 4-Dibenzothienylgruppe oder eine unsubstituierte 9-Phenanthrenylgruppe ist.

6. Organische Elektrolumineszenzvorrichtung, umfassend ein Elektrodenpaar und mindestens eine dazwischen angeordnete organische Schicht, wobei die organische Schicht die Arylaminverbindung nach einem der Ansprüche 1 bis 5 enthält.

7. Organische Elektrolumineszenzvorrichtung nach Anspruch 6, wobei die organische Schicht eine Lochtransportschicht ist.

8. Organische Elektrolumineszenzvorrichtung nach Anspruch 6, wobei die organische Schicht eine Elektronblockierungsschicht ist.

9. Organische Elektrolumineszenzvorrichtung nach Anspruch 6, wobei die organische Schicht eine Lochinjektionsschicht ist.

10. Organische Elektrolumineszenzvorrichtung nach Anspruch 6, wobei die organische Schicht eine lichtemittierende Schicht ist.

11. Elektronische Vorrichtung, umfassend ein Elektrodenpaar und mindestens eine dazwischen angeordnete organische Schicht, wobei die organische Schicht die Arylaminverbindung nach einem der Ansprüche 1 bis 5 enthält.

## Revendications

1. Composé arylamine de la formule générale (1) ou (2) suivante : 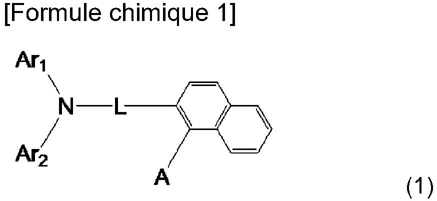
dans laquelle Ar₁ et Ar₂ peuvent être identiques ou différents et représentent un groupe phényle substitué ou non substitué, un groupe biphényle substitué ou non substitué, un groupe terphényle substitué ou non substitué, un groupe naphtyle substitué ou non substitué, un groupe anthracényle substitué ou non substitué, un groupe phénanthrényle substitué ou non substitué, un groupe indényle substitué ou non substitué, un groupe pyrényle substitué ou non substitué, un groupe pérylényle substitué ou non substitué, un groupe fluoranthényle substitué ou non substitué, un groupe triphénylényle substitué ou non substitué, un groupe pyridyle substitué ou non substitué, un groupe pyrimidinyle substitué ou non substitué, un groupe triazinyle substitué ou non substitué, un groupe furyle substitué ou non substitué, un groupe pyrrolyle substitué ou non substitué, un groupe thiényle substitué ou non substitué, un groupe quinolyle substitué ou non substitué, un groupe isoquinolyle substitué ou non substitué, un groupe benzofuranyle substitué ou non substitué, un groupe benzothiényle substitué ou non substitué, un groupe indolyle substitué ou non substitué, un groupe carbazolyle substitué ou non substitué, un groupe benzoxazolyle substitué ou non substitué, un groupe benzothiazolyle substitué ou non substitué, un groupe azafluorényle substitué ou non substitué, un groupe diazafluorényle substitué ou non substitué, un groupe azaspirobifluorényle substitué ou non substitué, un groupe diazaspirobifluorényle substitué ou non substitué, un groupe quinoxalinyle substitué ou non substitué, un groupe benzoimidazolyle substitué ou non substitué, un groupe pyrazolyle substitué ou non substitué, un groupe dibenzofuranyle substitué ou non substitué, un groupe dibenzothiényle substitué ou non substitué, un groupe naphtyridinyle substitué ou non substitué, un groupe phénanthrolinyle substitué ou non substitué, un groupe acridinyle substitué ou non substitué ou un groupe carbolinyle substitué ou non substitué,
lorsque Ar₁ et Ar₂ portent des substituants, les substituants sont un atome de deutérium, un groupe cyano, un groupe nitro, un atome d'halogène, un groupe silyle, un groupe alkyle linéaire ou ramifié comportant de 1 à 6 atomes de carbone, un groupe alkyloxy linéaire ou ramifié comportant de 1 à 6 atomes de carbone, un groupe alcényle, un groupe aryloxy, un groupe arylalkyloxy, un groupe phényle, un groupe biphényle, un groupe terphényle, un groupe naphtyle, un groupe anthracényle, un groupe phénanthrényle, un groupe indényle, un groupe pyrényle, un groupe pérylényle, un groupe fluoranthényle, un groupe triphénylényle ou un groupe hétérocyclique aromatique ;
L représente un groupe divalent substitué ou non substitué d'un hydrocarbure aromatique, un groupe divalent substitué ou non substitué d'un cycle hétérocyclique aromatique ou un groupe divalent substitué ou non substitué de composés aromatiques polycycliques condensés ;
A représente un groupe naphtyle substitué ou non substitué, un groupe dibenzofuranyle substitué ou non substitué, un groupe dibenzothiényle substitué ou non substitué, un groupe triphénylsilyle substitué ou non substitué ou un groupe phénanthrényle substitué ou non substitué, 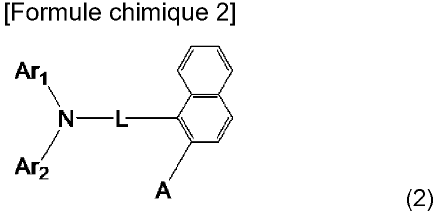
dans laquelle Ar₁, Ar₂, L et A sont tels que définis dans la formule générale (1).

2. Composé arylamine selon la revendication 1, dans lequel, dans la formule générale (1) ou (2), Ar₁ et/ou Ar₂ est un groupe phényle substitué ou non substitué, un groupe biphényle substitué ou non substitué, un groupe naphtyle substitué ou non substitué, un groupe dibenzofuranyle substitué ou non substitué, un groupe dibenzothiényle substitué ou non substitué ou un groupe phénanthrényle substitué ou non substitué.

3. Composé arylamine selon la revendication 1 ou 2, dans lequel, dans la formule générale (1) ou (2), L est un groupe phénylène non substitué, un groupe naphtylène non substitué ou un groupe biphénylène non substitué.

4. Composé arylamine selon l'une quelconque des revendications 1 à 3, dans lequel, dans la formule générale (1) ou (2), A est un groupe naphtyle non substitué, un groupe dibenzofuranyle non substitué, un groupe dibenzothiényle non substitué, un groupe triphénylsilyle non substitué ou un groupe phénanthrényle non substitué.

5. Composé arylamine selon l'une quelconque des revendications 1 à 4, dans lequel, dans la formule générale (1) ou (2), A est un groupe 1-naphtyle non substitué, un groupe 2-a-naphtyle non substitué, un groupe 1-dibenzofuranyle non substitué, un groupe 2-dibenzofuranyle non substitué, un groupe 3-dibenzofuranyle non substitué, un groupe 4-dibenzofuranyle non substitué, un groupe 3-dibenzothiényle non substitué, un groupe 4-dibenzothiényle non substitué ou un groupe 9-phénanthrényle non substitué.

6. Dispositif électroluminescent organique comprenant une paire d'électrodes et au moins une couche organique prise en sandwich entre celles-ci, dans lequel la couche organique comprend le composé arylamine selon l'une quelconque des revendications 1 à 5.

7. Dispositif électroluminescent organique selon la revendication 6, dans lequel la couche organique est une couche de transport de trous.

8. Dispositif électroluminescent organique selon la revendication 6, dans lequel la couche organique est une couche de blocage des électrons.

9. Dispositif électroluminescent organique selon la revendication 6, dans lequel la couche organique est une couche d'injection de trous.

10. Dispositif électroluminescent organique selon la revendication 6, dans lequel la couche organique est une couche émettrice de lumière.

11. Appareil électronique comprenant une paire d'électrodes et au moins une couche organique prise en sandwich entre celles-ci, dans lequel la couche organique comprend le composé arylamine selon l'une quelconque des revendications 1 à 5.
